(19) Europäisches Patentamt
European Patent Office
Office européen des brevets

(11) Veröffentlichungsnummer: **0 334 147**
**A1**

(12) # EUROPÄISCHE PATENTANMELDUNG

(21) Anmeldenummer: 89104381.2

(22) Anmeldetag: 13.03.89

(51) Int. Cl.4: C07D 207/337 , C07D 207/333 , A61K 31/40

(30) Priorität: 24.03.88 DE 3809860
03.10.88 IT 2215888

(43) Veröffentlichungstag der Anmeldung: 27.09.89 Patentblatt 89/39

(84) Benannte Vertragsstaaten: AT BE CH DE ES FR GB GR IT LI LU NL SE

(71) Anmelder: BAYER AG
D-5090 Leverkusen 1 Bayerwerk(DE)

(72) Erfinder: Hübsch, Walter, Dr.
Am Eckbusch 43/50
D-5600 Wuppertal 1(DE)
Erfinder: Angerbauer, Rolf, Dr.
Sterntalerweg 29
D-5600 Wuppertal 1(DE)
Erfinder: Fey, Peter, Dr.
Ursulastrasse 14
D-5600 Wuppertal 1(DE)
Erfinder: Bischoff, Hilmar, Dr.
Am Rohm 78
D-5600 Wuppertal 2(DE)
Erfinder: Petzinna, Dieter, Dr.
Peter-Berten-Strasse 10
D-4000 Düsseldorf 12(DE)
Erfinder: Schmidt, Delf, Dr.
Am Eckbusch 55b
D-5600 Wuppertal 1(DE)
Erfinder: Thomas, Günter, Dr.
Via Campogallo 21/14
I-20020 Arese (Mi)(IT)

(54) **Disubstituierte Pyrrole.**

(57) Disubstituierte Pyrrole der Formel

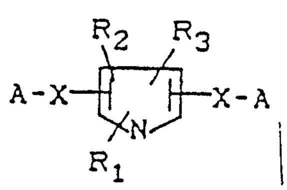

in welcher die Substituenten die in dem text angegebenen Bedeutungen haben und die als Wirkstoffe in Arzneimitteln eingesetzt werden können.

EP 0 334 147 A1

## Disubstituierte Pyrrole

Die Erfindung betrifft disubstituierte Pyrrole, Zwischenverbindungen zu ihrer Herstellung, ihre Herstellung und ihre Verwendung in Arzneimitteln.

Es ist bekannt, daß aus Pilzkulturen isolierte Lactonderivate Inhibitoren der 3-Hydroxy-3-methyl-glutaryl Coenzym A Reduktase (HMG-CoA-Reduktase) sind [Mevinolin, EP-A 22 478; US 4 231 938]. Darüberhinaus sind auch bestimmte Indolderivate bzw. Pyrazolderivate Inhibitoren der HMG-CoA-Reduktase [EP-A 1 114 027; US 4 613 610].

Es wurden nun disubstituierte Pyrrole der allgemeinen Formel (1),

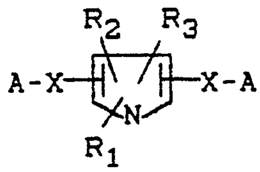

(I),

in welcher

$R^1$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,

worin

$R^4$, $R^5$ - gleich oder verschieden sind und Alkyl, Aryl, Aralkyl, Acyl, Alkylsulfonyl oder Arylsulfonyl bedeuten,

oder

- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,

worin

$R^4$ und $R^5$ die oben angegebene Bedeutung haben,

$R^2$ - Cycloalkyl bedeutet, oder

- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,

worin

$R^4$, $R^5$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^3$ - Wasserstoff bedeutet, oder

- Cycloalkyl bedeutet, oder

- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,

worin

$R^4$, $R^5$ die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

- Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,

worin
$R^4$ und $R^5$ die oben angegebene Bedeutung haben,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,
worin
$R^4$ und $R^5$ die oben angegebene Bedeutung haben,
X - eine Gruppe der Formel $-CH_2-CH_2-$ oder $-CH=CH-$ bedeutet,
und
A - eine Gruppe der Formel

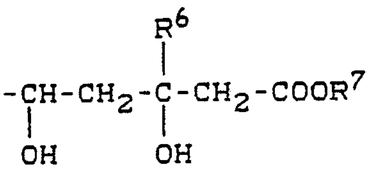

oder

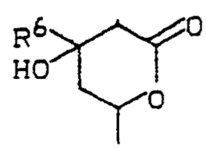

bedeutet,

worin
$R^6$ - für Wasserstoff oder Alkyl steht,
und
$R^7$ - Wasserstoff bedeutet oder
- für einen Alkyl, Aryl oder einen Aralkylrest oder
- für ein Kation steht,
gefunden.

Überraschenderweise zeigen die erfindungsgemäßen disubstituierten Pyrrole eine überlegene inhibitorische Wirkung auf die HMG-CoA Reduktase (3-Hydroxy-3-methylglutaryl Coenzym A Reduktase).

Cycloalkyl steht im allgemeinen für einen cyclischen Kohlenwasserstoffrest mit 3 bis 8 Kohlenstoffatomen. Bevorzugt ist der Cyclopropyl-, Cyclopentyl- und der Cyclohexylring. Beispielsweise seien Cyclopropyl, Cyclopentyl, Cyclohexyl, Cycloheptyl und Cyclooctyl genannt.

Alkyl steht im allgemeinen für einen geradkettigen oder verzweigten Kohlenwasserstoffrest mit 1 bis 12 Kohlenstoffatomen. Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Alkoxy steht im allgemeinen für einen über ein Sauerstoffatom gebundenen geradkettigen oder verzweigten Kohlenwasserstoffrest mit 1 bis 12 Kohlenstoffatomen. Bevorzugt ist Niederalkoxy mit 1 bis etwa 6 Kohlenstoffatomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Alkylthio steht im allgemeinen für einen über ein Schwefelatom gebundenen geradkettigen oder verzweigten Kohlenwasserstoffrest mit 1 bis 12 Kohlenstoffatomen. Bevorzugt ist Niederalkylthio mit 1 bis etwa 6 Kohlenstoffatomen. Besonders bevorzugt ist ein Alkylthiorest mit 1 bis 4 Kohlenstoffatomen. Beispielsweise seien Methylthio, Ethylthio, Propylthio, Isopropylthio, Butylthio, Isobutylthio, Pentylthio, Isopentylthio, Hexylthio, Isohexylthio, Heptylthio, Isoheptylthio, Octylthio oder Isooctylthio genannt.

Alkylsulfonyl steht im allgemeinen für einen geradkettigen oder verzweigten Kohlenwasserstoffrest mit 1 bis 12 Kohlenstoffatomen, der über eine $SO_2$-Gruppe gebunden ist. Bevorzugt ist Niederalkylsulfonyl mit 1 bis etwa 6 Kohlenstoffatomen. Beispielsweise seien genannt: Methylsulfonyl, Ethylsulfonyl, Propylsulfonyl, Isopropylsulfonyl, Butylsulfonyl, Isobutylsulfonyl, Pentylsulfonyl, Isopentylsulfonyl, Hexylsulfonyl, Isohexylsulfonyl.

Sulfamoyl (Aminosulfonyl) steht für die Gruppe $-SO_2-NH_2$.

Aryl steht im allgemeinen für einen aromatischen Rest mit 6 bis etwa 12 Kohlenstoffatomen. Bevorzugte Arylreste sind Phenyl, Naphthyl und Biphenyl, insbesondere Phenyl.

Aryloxy steht im allgemeinen für einen aromatischen Rest mit 6 bis etwa 12 Kohlenstoffatomen, der über ein Sauerstoffatom gebunden ist. Bevorzugte Aryloxyreste sind Phenoxy oder Naphthyloxy, insbesondere Phenoxy.

Arylthio steht im allgemeinen für einen aromatischen Rest mit 6 bis etwa 12 Kohlenstoffatomen, der

über ein Schwefelatom gebunden ist. Bevorzugte Arylthioreste sind Phenylthio oder Naphthylthio, insbesondere Phenylthio.

Arylsulfonyl steht im allgemeinen für einen aromatischen Rest mit 6 bis etwa 12 Kohlenstoffatomen, der über eine $SO_2$-Gruppe gebunden ist. Beispielsweise seien genannt: Phenylsulfonyl, Naphthylsulfonyl und Biphenylsulfonyl, insbesondere Phenylsulfonyl.

Aralkyl steht im allgemeinen für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatome im aromatischen Teil. Beispielsweise seien folgende Aralkylreste genannt: Benzyl, Naphthylmethyl, Phenethyl und Phenylpropyl, insbesondere Benzyl.

Aralkoxy steht im allgemeinen für einen Aralkylrest mit 7 bis 14 Kohlenstoffatomen, wobei die Alkylenkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt: Benzyloxy, Naphthylmethoxy, Phenethoxy und Phenylpropoxy, insbesondere Benzyloxy.

Aralkylthio steht im allgemeinen für einen Aralkylrest mit 7 bis etwa 14 Kohlenstoffatomen wobei die Alkylkette über ein Schwefelatom gebunden ist. Bevorzugt werden Aralkylthioreste mit 1 bis 6 Kohlenstoffatomen im aliphatischen Teil und 6 bis 12 Kohlenstoffatomen im aromatischen Teil, Beispielsweise seien folgende Aralkylthioreste genannt: Benzylthio, Naphthylmethylthio, Phenethylthio und Phenylpropylthio, insbesondere Benzylthio.

Aralkylsulfonyl steht im allgemeinen für einen Aralkylrest mit 7 bis etwa 14 Kohlenstoffatomen, wobei die Alkylreste über eine $SO_2$-Kette gebunden ist. Bevorzugt werden Aralkylsulfonylreste mit 1 bis 6 Kohlenstoffatomen im aliphatischen Teil und 6 bis 12 Kohlenstoffatomen im aromatischen Teil. Beispielsweise seien folgende Aralkylsulfonylreste genannt: Benzylsulfonyl, Naphthylmethylsulfonyl, Phenethlysulfonyl und Phenylpropylsulfonyl, insbesondere Benzylsulfonyl.

Alkoxycarbonyl kann beispielsweise durch die Formel

$$-\underset{\overset{\|}{O}}{C}-OAlkyl$$

dargestellt werden, Alkyl steht hierbei für einen geradkettigen oder verzweigten Kohlenwasserstoffrest mit 1 bis 12 Kohlenstoffatomen. Bevorzugt wird Niederalkoxycarbonyl mit 1 bis etwa 6 Kohlenstoffatomen im Alkylteil. Insbesondere bevorzugt wird ein Alkoxycarbonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Acyl steht im allgemeinen für Phenyl oder geradkettiges oder verzweigtes Niederalkyl mit 1 bis etwa 6 Kohlenstoffatomen, die über eine Carbonylgruppe gebunden sind. Bevorzugt sind Phenyl und Alkylreste mit bis zu 4 Kohlenstoffatomen. Beispielsweise seien genannt: Benzoyl, Acetyl, Ethylcarbonyl, Propylcarbonyl, Isopropylcarbonyl, Butylcarbonyl und Isobutylcarbonyl, insbesondere Benzyl und Acetyl.

Halogen steht im allgemeinen für Fluor, Chlor, Brom oder Iod, bevorzugt für Fluor, Chlor oder Brom. Besonders bevorzugt steht Halogen für Fluor oder Chlor.

Heteroaryl steht im allgemeinen für einen 5- bis 6-gliedrigen aromatischen Ring, der als Heteroatome Sauerstoff, Schwefel und/oder Stickstoff enthalten kann und an den weitere aromatische Ringe ankondensiert sein können. Bevorzugt sind 5- und 6-gliedrige aromatische Ringe, die einen Sauerstoff, ein Schwefel und/oder bis zu 2 Stickstoffatome enthalten und die gegebenenfalls benzokondensiert sind. Als besonders bevorzugte Heteroarylreste seien genannt: Thienyl, Furyl, Pyrolyl, Pyrazolyl, Pyridyl, Pyrimidyl, Pyrazinyl, Pyridazinyl, Chinolyl, Isochinolyl, Chinazolyl, Chinoxalyl, Phthalazinyl, Cinnolyl, Thiazolyl, Benzothiazolyl, Isothiazolyl, Oxazolyl, Benzoxazolyl, Isoxazolyl, Imidazolyl, Benzimidazolyl, Indolyl, und Isoindolyl.

Falls $R^7$ für einen Alkyl, Aryl oder Aralkylrest steht, bildet sich eine Estergruppe. Bevorzugt wurden physiologisch verträgliche Ester, die in vivo leicht zu einer freien Carboxylgruppe und einem entsprechenden physiologisch verträglichen Alkohol hydrolysiert wurden. Hierzu gehören beispielsweise Alkylester ($C_1$ bis $C_4$) und Aralkylester ($C_7$ bis $C_{10}$), bevorzugt Niederalkylester ($C_1$ bis $C_4$) sowie Benzylester. Insbesondere bevorzugt seien die folgenden Esterreste genannt: Methylester, Ethylester, Propylester, Benzylester.

$R^7$ kann auch für ein Kation ($M^{n+}$, wobei n die Wertigkeit bedeutet), bevorzugt ein physiologisch verträgliches Metall- oder Ammoniumkation stehen. Insbesondere bevorzugt sind hierbei Alkali- bzw. Erdalkalikationen wie beispielsweise Natrium, Kalium, Magnesium- oder Calciumkationen, sowie Aluminium- oder Ammoniumkationen, sowie nicht-toxische substituierte Ammoniumkationen aus Aminen wie Diniedrigalkylamine ($C_1$ bis etwa $C_6$), Triniedrigalkylamine ($C_1$ bis etwa $C_6$), Dibenzylamin, N,N′-Dibenzylethylendiamin, N-Benzyl-β-phenylethylamin, N-Methylmorpholin oder N-Ethylmorpholin, Dihydroabiethylamin, N,N′-dihydroabiethylethylendiamin, N-Niedrigalkylpiperidin und andere Amine, die zur Bildung von Salzen verwendet werden können.

Im Rahmen der vorliegenden Erfindung können die disubstituierten Pyrrole durch die allgemeinen Formeln (Ia-f)

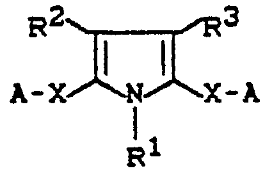 (Ia)

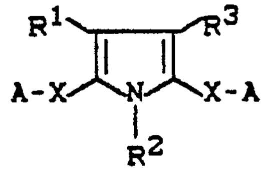 (Ib)

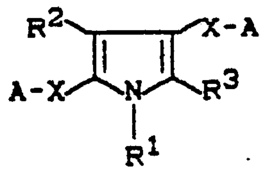 (Ic)

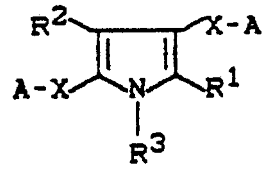 (Id)

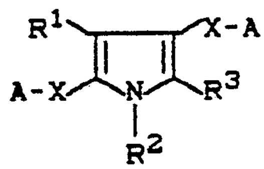 (Ie)

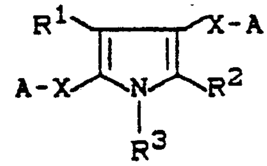 (If)

in welcher $R^1$, $R^2$, $R^3$, X und A die oben angegebene Bedeutung haben, dargestellt werden.

Bevorzugt sind Verbindungen der allgemeinen Formeln (I) in welchen

$R^1$ - Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder ver schieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder

- Phenyl oder Naphthyl bedeutet, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^4R^5$,

wobei

$R^4$ und $R^5$ gleich oder verschieden sind und Niederalkyl, Phenyl, Benzyl, Acetyl, Benzoyl, Phenylsulfonyl oder Niederalkylsulfonyl bedeuten,

$R^2$ - Cyclopropyl, Cyclopentyl oder Cyclohexyl bedeutet, oder

- Niederalkyl bedeutet, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^4R^5$,

worin

$R^4$ und $R^5$ die oben angegebene Bedeutung haben, oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,

$R^3$ - Wasserstoff, oder

- Cyclopropyl, Cyclopentyl oder Cyclohexyl bedeutet, oder

- Niederalkyl bedeutet, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl oder durch eine Gruppe der Formel $-NR^4R^5$,

worin

$R^4$ und $R^5$ die oben angegebene Bedeutung haben, oder durch Pyridyl, Pyrimidyl, Pyrazinyl, Pyridazinyl,

Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, Niederalkyl, Niederalkoxy, Trifluormethyl, oder Trifluormethoxy substituiert sein können,

- Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Chinolinyl, Benzothiazolyl, Benzoxazolyl oder Benzimidazolyl bedeutet, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
- Phenyl oder Naphthyl bedeutet, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^4R^5$,

wobei

$R^4$ und $R^5$ die oben angegebene Bedeutung haben,

X - eine Gruppe der Formel -CH=CH- bedeutet

und

A - eine Gruppe der Formel

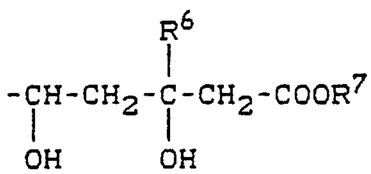 oder 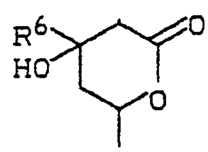 bedeutet,

worin

$R^6$ - Wasserstoff oder Niederalkyl bedeutet,

und

$R^7$ - einen $C_1$ bis $C_6$ Alkylrest, ein $C_6$ bis $C_{12}$ Arylrest oder ein $C_7$ bis $C_{10}$ Aralkylrest bedeutet, oder

- ein physiologisch verträgliches Kation bedeutet.

Besonders bevorzugt sind Verbindungen der allgemeinen Formel I

in welcher

$R^1$ - Pyridyl, Pyrimidyl, Chinolyl oder Isochinolyl bedeutet, das durch Fluor, Chlor, Methyl, Methoxy oder Trifluormethyl substituiert sein kann, oder

- Phenyl bedeutet, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,

$R^2$ - Cyclopropyl, Cyclopentyl oder Cyclohexyl bedeutet, oder

- Methyl, Ethyl, Propyl, Isopropyl, Butyl, sec.-Butyl oder tert.Butyl bedeutet, das substituiert sein kann durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,

$R^3$ - Wasserstoff, Cyclopropyl, Cyclopentyl oder Cyclohexyl bedeutet, oder

- Methyl, Ethyl, Propyl, Isopropyl, Butyl, Isobutyl, tert.Butyl, Pentyl, Isopentyl, Hexyl oder Isohexyl bedeutet, das substituiert sein kann durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe $-NR^4R^5$,

wobei

$R^4$ und $R^5$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,

oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.Butoxy, Trifluormethyl oder Trifluormethoxy substituiert sein können, oder

- Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl bedeutet, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder

- Phenyl bedeutet, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbo nyl, Butoxycarbonyl, Isobutoxycarbonyl, tert.Butoxycarbonyl oder durch eine Gruppe $-NR^4R^5$ substituiert sein kann,

wobei

$R^4$ und $R^5$ die oben angegebene Bedeutung haben,

X - eine Gruppe der Formel $-CH=CH-$ bedeutet

und

A - eine Gruppe der Formel

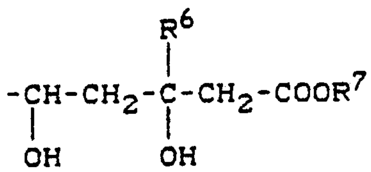 oder 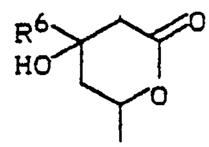 bedeutet,

worin

$R^6$ - Wasserstoff, Methyl, Ethyl, Propyl, Isopropyl, Butyl, Isobutyl oder tert.Butyl bedeutet

und

$R^7$ - Wasserstoff, Methyl, Ethyl, Propyl, Isopropyl, Butyl, Isobutyl, tert.Butyl oder Benzyl bedeutet, oder

- ein Natrium-, Kalium-, Calcium- oder Magnesium- oder Ammoniumion bedeutet.

Ganz besonders bevorzugt sind Verbindungen der allgemeinen Formeln I

in welcher

$R^1$ - Phenyl bedeutet, das bis zu 2-fach gleich oder verschieden durch Methyl, Phenoxy, Fluor, oder Trifluormethyl substituiert sein kann,

$R^2$ - Isopropyl, Cyclopropyl oder tert.Butyl bedeutet,

$R^3$ Cyclopropyl, Isopropyl, tert.-Butyl oder Phenyl bedeutet, das bis zu zweifach gleich oder verschieden durch Fluor, Methyl, Phenoxy oder Trifluormethyl substituiert sein kann,

X - eine Gruppe der Formel

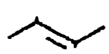 (E-konfiguriert) bedeutet

und

A - eine Gruppe der Formel

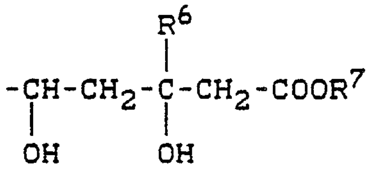 oder 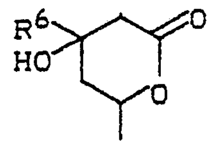 bedeutet,

worin
$R^6$ - Wasserstoff bedeutet
und
$R^7$ - Wasserstoff, Methyl oder Ethyl bedeutet, oder
- ein Natrium- oder Kaliumkation bedeutet.

Die erfindungsgemäßen disubstituierten Pyrrole der allgemeinen Formel (I) haben mehrere asymmetrische Kohlenstoffatome und können daher in verschiedenen stereochemischen Formen existieren. Sowohl die einzelnen Isomeren als auch deren Mischungen sind Gegenstand der Erfindung.

Besonders bevorzugt sind die Isomeren der allgemeinen Formel (I), die in 2,5 und 3,5-Position mit dem Rest X-A substituiert sind.

Je nach der Bedeutung der Gruppen X bzw. der Reste A ergeben sich unterschiedliche Stereoisomere, die im folgenden näher erläutert werden sollten:

a) Steht die Gruppe -X- für eine Gruppe der Formel -CH=CH- , so können die erfindungsgemäßen Verbindungen in zwei stereoisomeren Formen existieren, die an der Doppelbindung E-konfiguriert (II) oder Z-konfiguriert (III) sein können:

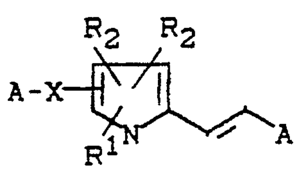 (II) E-Form

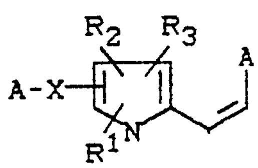 (III) Z-Form

bevorzugt sind diejenigen Verbindungen der allgemeinen Formel (I), die E-konfiguiert sind (II).

$R^1$, $R^2$, $R^3$, X und A haben die oben angegebene Bedeutung.

b) Steht der Rest -A- für eine Gruppe der Formel

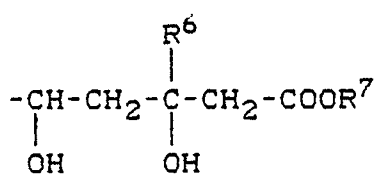

so besitzen die Verbindungen der allgemeinen Formel (I) mindestens zwei asymmetrische Kohlenstoffatome, nämlich die beiden Kohlenstoffatome an denen die Hydroxygruppen gebunden sind. Je nach der relativen Stellung dieser Hydroxygruppen zueinander, können die erfindungsgemäßen Verbindungen in der erythro-Konfiguration (IV) oder in der threo-Konfiguration (V) vorliegen.

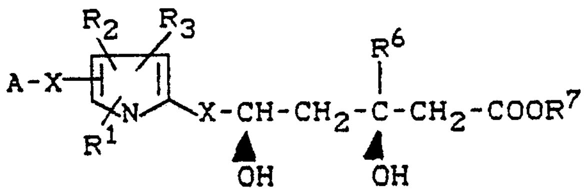

erythro-Form (IV)

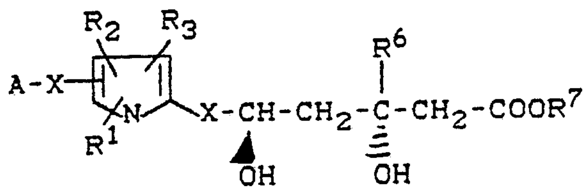

threo-Form (V)

Sowohl von den Verbindungen der erythro- als auch der threo-Konfiguration existieren wiederum jeweils zwei Enantiomere, nämlich 3R,5S-Isomeres bzw. 3S,5R-Isomeres (erythro-Form) sowie 3R,5R-Isomeres und 3S,5S-Isomeres (threo-Form).

Bevorzugt sind hierbei die erythro-konfigurierten Isomeren, besonders bevorzugt das 3R,5S-Isomere sowie das 3R,5S-3S,5R-Racemat.

c) Steht der Rest -A für eine Gruppe der Formel

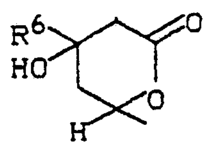

so besitzen die disubstituierten Pyrrole mindestens zwei asymmetrische Kohlenstoffatome, nämlich das Kohlenstoffatom an dem die Hydroxygruppe gebunden ist, und die Kohlenstoffatome an welchem der Rest

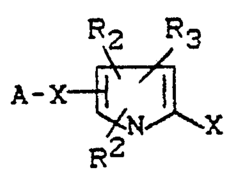

gebunden ist. Je nach der Stellung der Hydroxygruppe zur freien Valenz am Lactonring können die disubstituierten Pyrrole als cis-Lactone (VI) oder als trans-Lactone (VII) vorliegen.

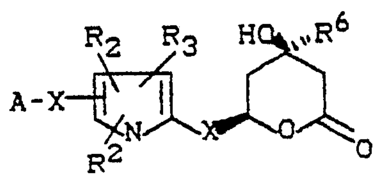

cis-Lacton (VI)

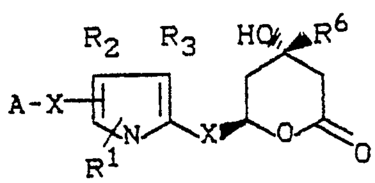

trans-Lacton (VII)

Sowohl vom cis-Lacton aus als auch vom trans-Lacton existieren wiederum jeweils zwei Isomere

nämlich das 4R,6R-Isomere bzw. das 4S,6S-Isomere (cis-Lacton), sowie das 4R,6S-Isomeren bzw. 4S,6R-Isomere (trans-Lacton). Bevorzugte Isomere sind die trans-Lactone. Besonders bevorzugt ist hierbei das 4R,6S-Isomere (trans) sowie das 4R,6S-4S,6R-Racemat.

Beispielsweise seien die folgenden isomeren Formen der substituierten Pyrrole genannt:

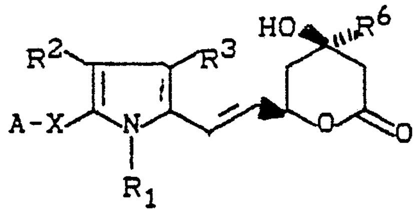

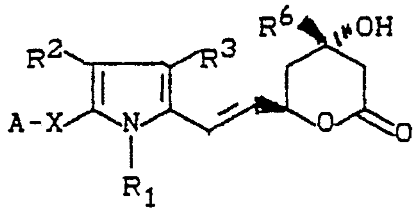

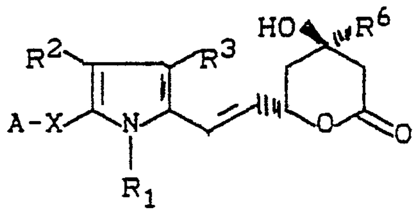

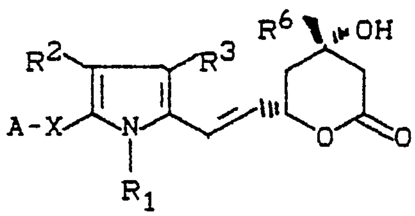

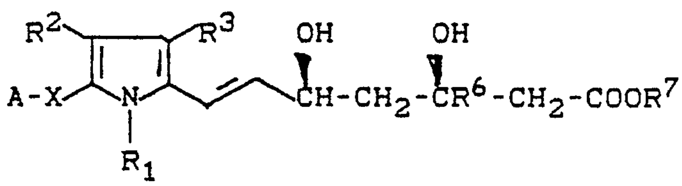

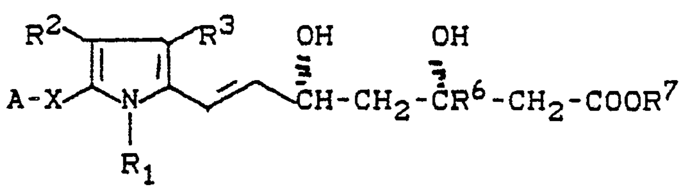

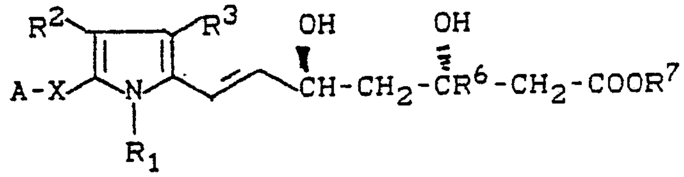

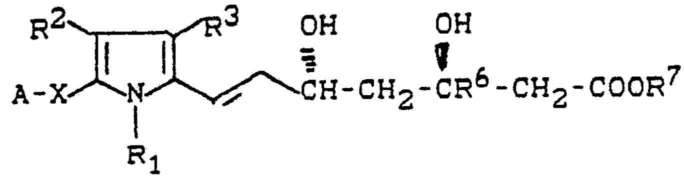

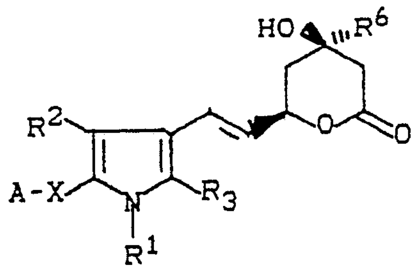

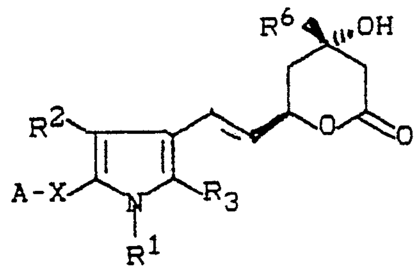

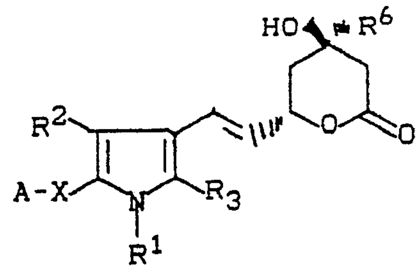

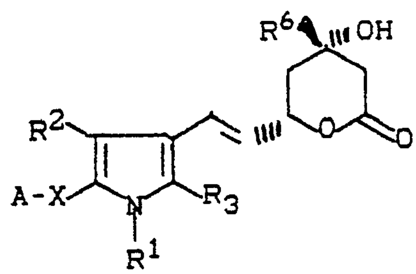

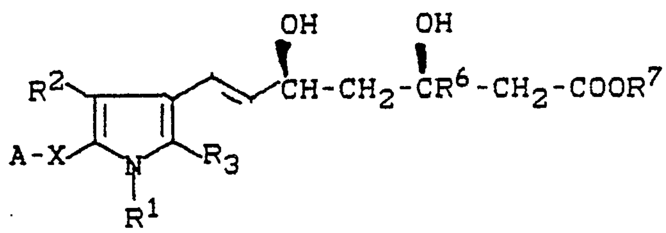

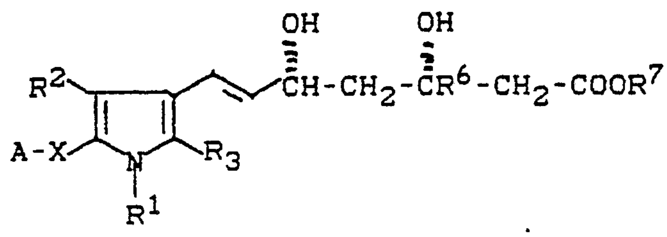

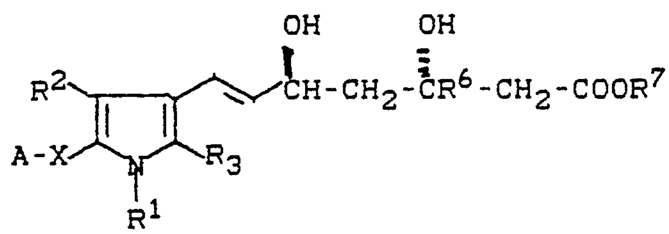

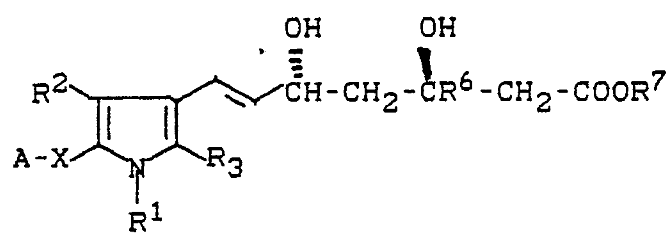

Darüber hinaus ergeben sich weitere Möglichkeiten der Isomerenbildung, da die erfindungsgmäßen disubstituierten Pyrrole durch zwei Gruppen der Formel -X-A substituiert sind. Auch für die zweite Gruppe -X-A im Molekül gilt das oben gesagte. Es sind ebenso alle Stereoisomeren Gegenstand der Erfindung, die durch die zweite Gruppe der Formel -X-A, insbesondere im Zusammenhang mit der ersten Gruppe -X-A entstehen.

Darüber hinaus ergeben sich weitere Möglichkeiten, da die erfindungsgemäßen disubstituierten Pyrrole durch die Variation bezüglich der Stellung der Reste $R_1$, $R_2$ und $R^3$ gekennzeichnet sind,

Außerdem wurde ein Verfahren zur Herstellung der disubstituierten Pyrrole der allgemeinen Formel (I)

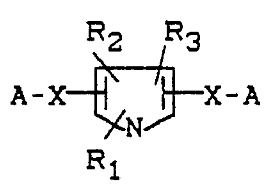

(I)

in welcher

$R^1$, $R^2$, $R^3$ und A und X die oben angegebene Bedeutung haben,

gefunden,

das dadurch gekennzeichnet ist, daß man Ketone der allgemeinen Formel (VIII)

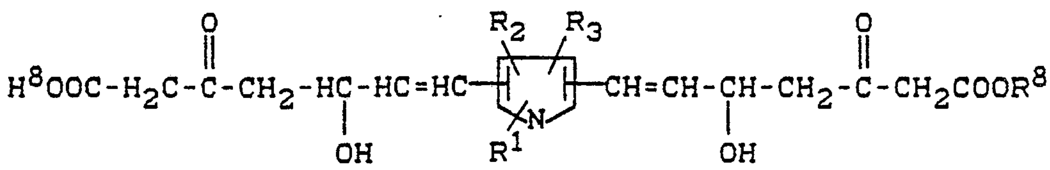

(VIII)

in welcher
$R^1$, $R^2$ und $R^3$ die oben angegebene Bedeutung haben,
und
$R^8$ - für Alkyl steht,
reduziert,
im Fall der Herstellung der Säuren die Ester verseift,
im Fall der Herstellung der Lactone die Carbonsäuren cyclisiert,
im Fall der Herstellung der Salze entweder die Ester oder die Lactone verseift,
im Fall der Herstellung der Ethylenverbindungen ($X = -CH_2-CH_2-$) die Ethenverbindungen ($X = -CH=CH-$) nach üblichen Methoden hydriert,
und gegebenenfalls Isomeren trennt.

Das erfindungsgemäße Verfahren kann durch das folgendes Formelschema erläutert werden:

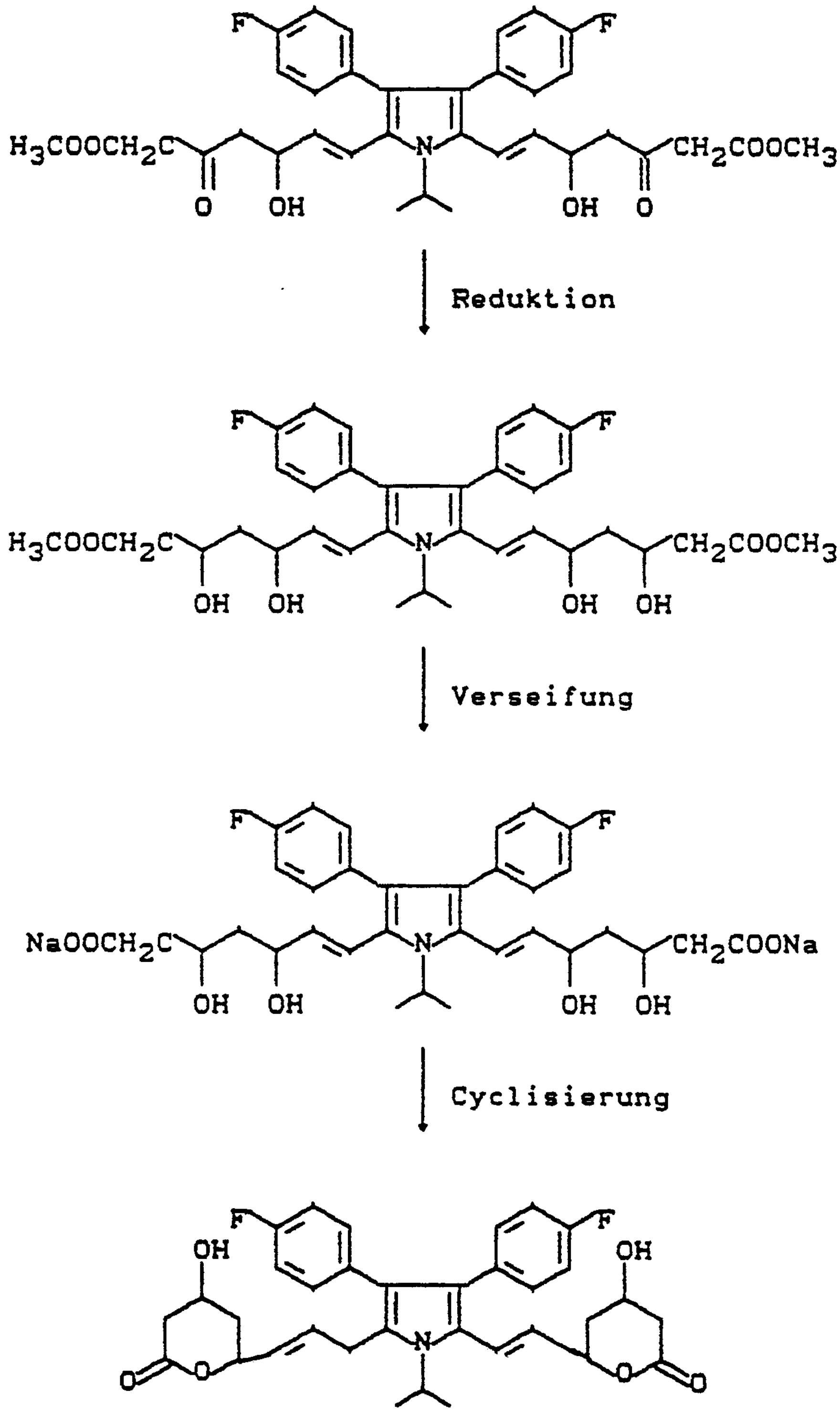
Reduktion
Verseifung
Cyclisierung

oder

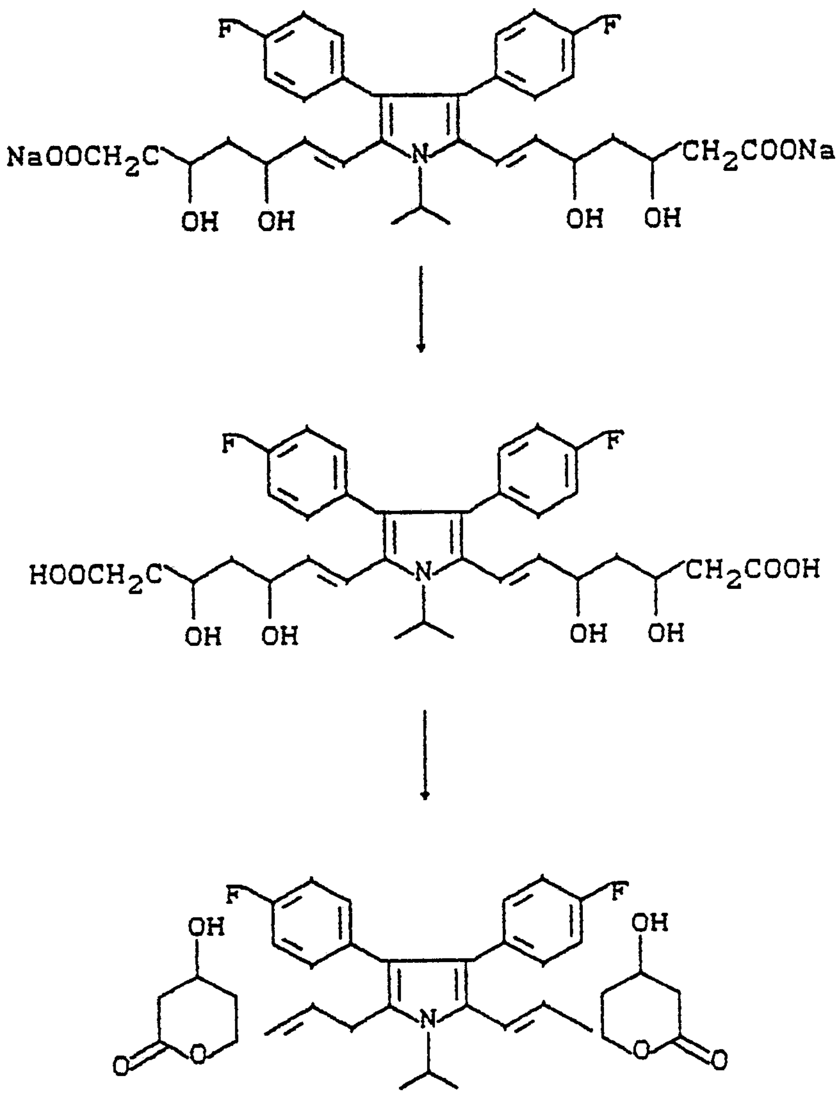

Die Reduktion kann mit den üblichen Reduktionsmitteln, bevorzugt mit solchen, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkyl-hydrido-boranaten, Natrium-trialkyl-hydrido-boranaten, Natrium-cyano-trihydrido-boranat oder Lithiumaluminiumhydrid durchgeführt. Ganz besonders bevorzugt wird die Reduktion mit Natriumborhydrid, in Anwesenheit von Triethylboran durchgeführt.

Als Lösemittel eignen sich hierbei die üblichen organischen Lösemittel, die sich unter den Reaktionsbedingungen nicht verändern. Hierzu gehören bevorzugt Ether wie beispielsweise Diethylether, Dioxan, Tetrahydrofuran oder Dimethoxyethan, oder Halogenkohlenwasserstoffe wie beispielsweise Dichlormethan, Trichlormethan, Tetrachlormethan, 1,2-Dichlorethan, oder Kohlenwasserstoffe wie beispielsweise Benzol, Toluol oder Xylol. Ebenso ist es möglich Gemische der genannten Lösemittel einzusetzen.

Besonders bevorzugt wird die Reduktion der Ketongruppe zur Hydroxygruppe unter Bedingungen durchgeführt, bei denen die übrigen funktionellen Gruppen wie beispiels weise die Alkoxycarbonylgruppe nicht verändert werden. Hierzu besonders geeignet ist die Verwendung von Natriumborhydrid als Reduktionsmittel, in Anwesenheit von Triethylboran in inerten Lösemitteln wie vorzugsweise Ethern.

Die Reduktion erfolgt im allgemeinen in einem Temperaturbereich von -90 °C bis +30 °C, bevorzugt von -80 °C bis 0 °C.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 2 mol, bevorzugt von 1 bis 1,5 mol bezogen auf 1 mol der Ketoverbindung eingesetzt.

Unter den oben angegebenen Reaktionsbedingungen wird im allgemeinen die Carbonylgruppe zur Hydroxygruppe reduziert, ohne daß Reduktion an der Doppelbindung zur Einfachbindung erfolgt.

Zur Herstellung von Verbindungen der allgemeinen Formel (I), in welchen X für eine Ethylengruppierung steht, kann die Reduktion der Ketone (VIII) unter solchen Bedingungen durchgeführt werden, unter denen sowohl die Carbonylgruppe als auch die Doppelbindung reduziert wird.

Darüber hinaus ist es auch möglich, die Reduktion der Carbonylgruppe und die Reduktion der Doppelbindung in zwei getrennten Schritten durchzuführen.

Die Carbonsäuren im Rahmen der allgemeinen Formel (I) entsprechen der Formel (Ig)

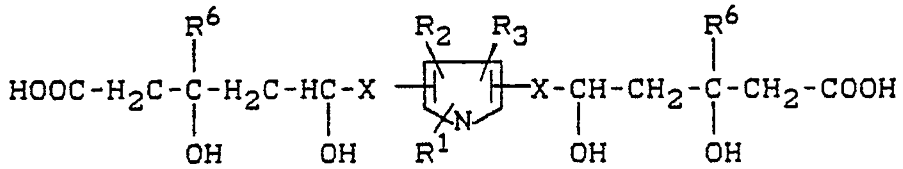

(Ig)

in welcher

$R^1$, $R^2$, $R^3$, $R^6$ und X die oben angegebene Bedeutung haben.

Die Carbonsäureester im Rahmen der allgemeinen Formel (I) entsprechen der Formel (Ih)

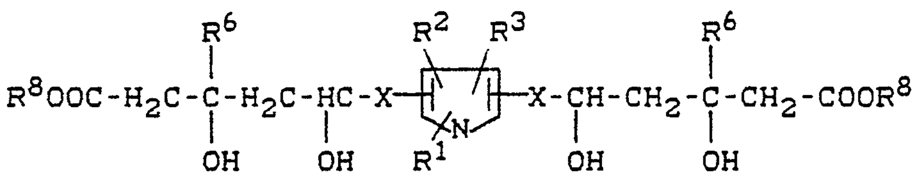

(Ih)

in welcher

$R^1$, $R^2$, $R^3$, $R^6$ und X die oben angegebene Bedeutung haben,

und

$R^8$ - für Alkyl steht.

Die Salze der erfindungsgemäßen Verbindung im Rahmen der allgemeinen Formel (I) entsprechen der Formel (Ii)

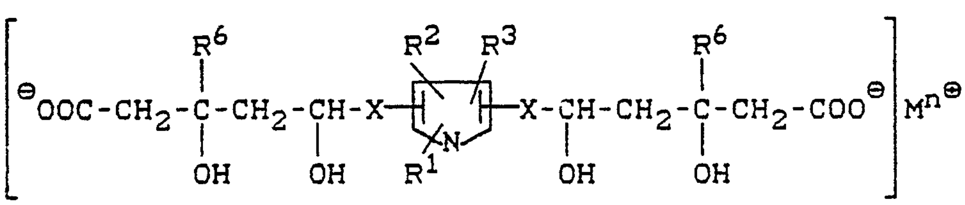

(Ii)

in welcher

$R^1$, $R^2$, $R^3$, $R^6$ und X die oben angegebene Bedeutung haben,

und

$M^{n\oplus}$ für ein n wertiges Kation steht.

Die Lactone im Rahmen der allgemeinen Formel (I) entsprechen der Formel (Ij)

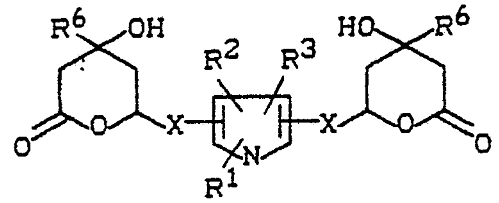

(Ij)

in welcher
$R^1$, $R^2$, $R^3$, $R^6$ und X die oben angegebene Bedeutung haben.

Zur Herstellung der erfindungsgemäßen Carbonsäuren der allgemeinen Formel (Ig) werden im allgemeinen die Carbonsäureester der allgemeinen Formel (Ih) oder die Lactone der allgemeinen Formel (Ij) nach üblichen Methoden verseift. Die Verseifung erfolgt im allgemeinen indem man die Ester oder die Lactone in inerten Lösemitteln mit üblichen Basen behandelt, wobei im allgemeinen zunächst die Salze der allgemeinen Formel (Ii) entstehen, die anschließend in einem zweiten Schritt durch Behandeln mit Säure in die freien Säuren der allgemeinen Formel (Ig) überführt werden konnen.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters bzw. des Lactons eingesetzt. Besonders bevorzugt verwendet man molare Mengen der Reaktanden.

Bei der Durchführung der Reaktion entstehen im ersten Schritt die Salze der erfindungsgemäßen Verbindungen (Ii) als Zwischenprodukte, die isoliert werden können. Die erfindungsgemäßen Säuren (Ig) erhält man durch Behandeln der Salze (Ii) mit üblichen anorganischen Säu ren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren (Ig) hierbei als vorteilhaft erwiesen, die basische Reaktionsmischung der Verseifung in einem zweiten Schritt ohne Isolierung der Salze anzusäuern. Die Säuren können dann in üblicher Weise isoliert werden.

Zur Herstellung der erfindungsgemäßen Lactone der Formel (Ij) werden im allgemeinen die erfindungsgemäßen Carbonsäuren (Ig) nach üblichen Methoden cyclisiert, beispielsweise durch Erhitzen der entsprechenden Säure in inerten organischen Lösemitteln, gegebenenfalls in Anwesenheit von Molsieb.

Als Lösemittel eignen sich hierbei Kohlenwasserstoffe wie Benzol, Toluol, Xylol, Erdölfraktionen, oder Tetralin oder Diglyme oder Triglyme. Bevorzugt werden Benzol, Toluol oder Xylol eingesetzt. Ebenso ist es möglich Gemische der genannten Lösemittel einzusetzen. Besonders bevorzugt verwendet man Kohlenwasserstoffe, insbesondere Toluol, in Anwesenheit von Molsieb.

Die Cyclisierung wird im allgemeinen in einem Temperaturbereich von -40 °C bis +200 °C, bevorzugt von -25 °C bis +50 °C durchgeführt.

Die Cyclisierung wird im allgemeinen bei Normaldruck durchgeführt, es ist aber auch möglich, das Verfahren bei Unterdruck oder bei Überdruck durchzuführen (z.B. in einem Bereich von 0,5 bis 5 bar).

Darüberhinaus wird die Cyclisierung auch in inerten organischen Lösemitteln, mit Hilfe von cyclisierenden bzw. wasserabspaltenden Agentien durchgeführt. Als wasserabspaltende Agentien werden hierbei bevorzugt Carbodiimide verwendet. Als Carbodiimide werden bevorzugt N,N′-Dicyclohexylcarbodiimid-Paratoluolsulfonat, N-Cyclohexyl-N′-[2-(N″-methylmorpholinium)ethyl]carbodiimid oder N-(3-Dimethylaminopropyl)-N′-ethylcarbodiimid-Hydrochlorid eingesetzt.

Als Lösemittel eignen sich hierbei die üblichen organischen Lösemittel. Hierzu gehören bevorzugt Ether wie Diethylether, Tetrahydrofuran oder Dioxan, oder Chlorkohlenwasserstoffe wie Methylenchlorid, Chloroform oder Tetrachlorkohlenstoff, oder Kohlenwasserstoffe wie Benzol, Toluol, Xylol oder Erdölfraktionen. Besonders bevorzugt werden Chlorkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen. Besonders bevorzugt werden Chlorkohlenwasserstoffe wie beispielsweise Methylenchlorid, Chloroform oder Tetrachlorkohlenstoff eingesetzt.

Die Reaktion wird im allgemeinen in einem Temperaturbereich von 0°C bis +80°C, bevorzugt von +10°C bis +50°C durchgeführt.

Bei der Durchführung der Cyclisierung hat es sich als vorteilhaft erwiesen, die Cyclisierungsmethode mit Hilfe von Carbodiimiden als dehydratisierenden Agentien einzusetzen.

Die Trennung der Isomeren in die stereoisomer einheitlichen Bestandteile erfolgt im allgemeinen nach üblichen Methoden wie sie beispielsweise von E.L. Eliel, Stereochemistry of Carbon Compounds, McGraw Hill, 1962 beschrieben wird. Bevorzugt wird hierbei die Trennung der Isomeren auf der Stufe der racemischen Lactone. Besonders bevorzugt wird hierbei die racemische Mischung der trans-Lactone (VII) durch Behandeln entweder mit D-(+)-oder L-(-)-α-Methylbenzylamin nach üblichen Methoden in die diastereomeren Dihydroxyamide (Ik)

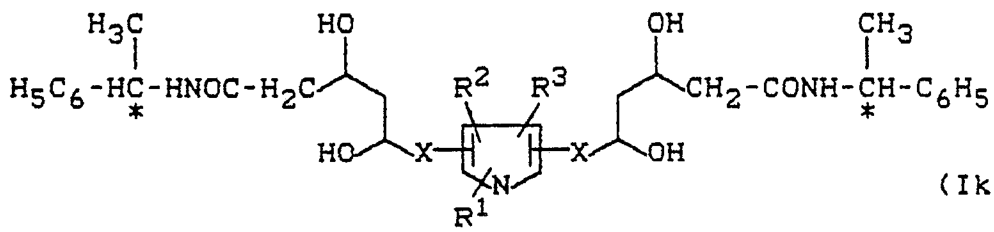

(Ik)

überführt, die anschließend wie üblich durch Chromatographie oder Kristallisation in die einzelnen Diastereomeren getrennt werden können. Anschließende Hydrolyse der reinen diastereomeren Amide nach üblichen Methoden, beispielsweise durch Behandeln der diastereomeren Amide mit anorganischen Basen wie Natriumhydroxid oder Kalium hydroxid in Wasser und/oder organischen Lösemitteln wie Alkoholen z.B. Methanol, Ethanol, Propanol oder Isopropanol, ergeben die entsprechenden enantiomerenreinen Dihydroxysäuren (Ig), die wie oben beschrieben durch Cyclisierung in die enantiomerenreinen Lactone überführt werden können. Im allgemeinen gilt für die Herstellung der erfindungsgemäßen Verbindungen der allgemeinen Formel (I) in enantiomerenreiner Form, daß die Konfiguration der Endprodukte nach der oben beschriebenen Methode abhängig ist von der Konfiguration der Ausgangsstoffe.

Die Isomerentrennung soll im folgenden Schema beispielhaft erläutert werden:

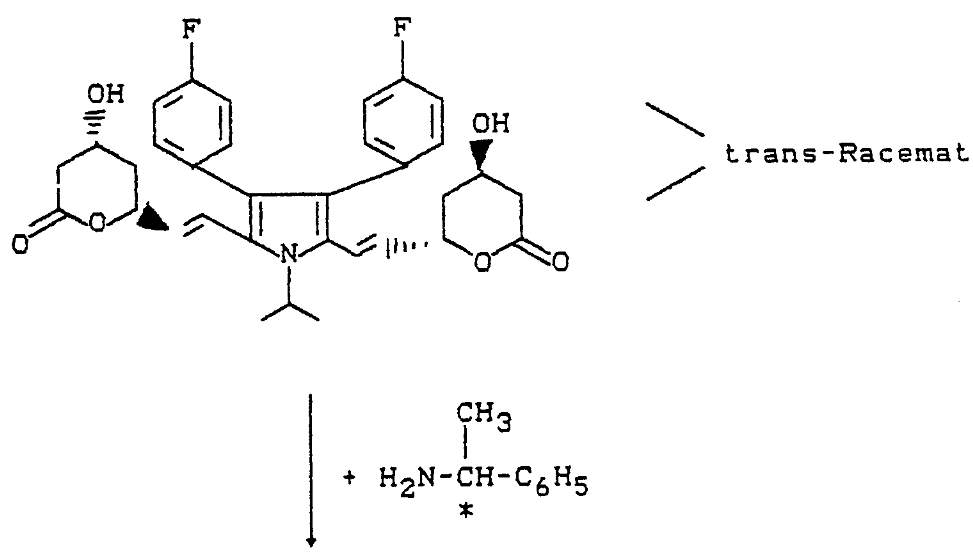

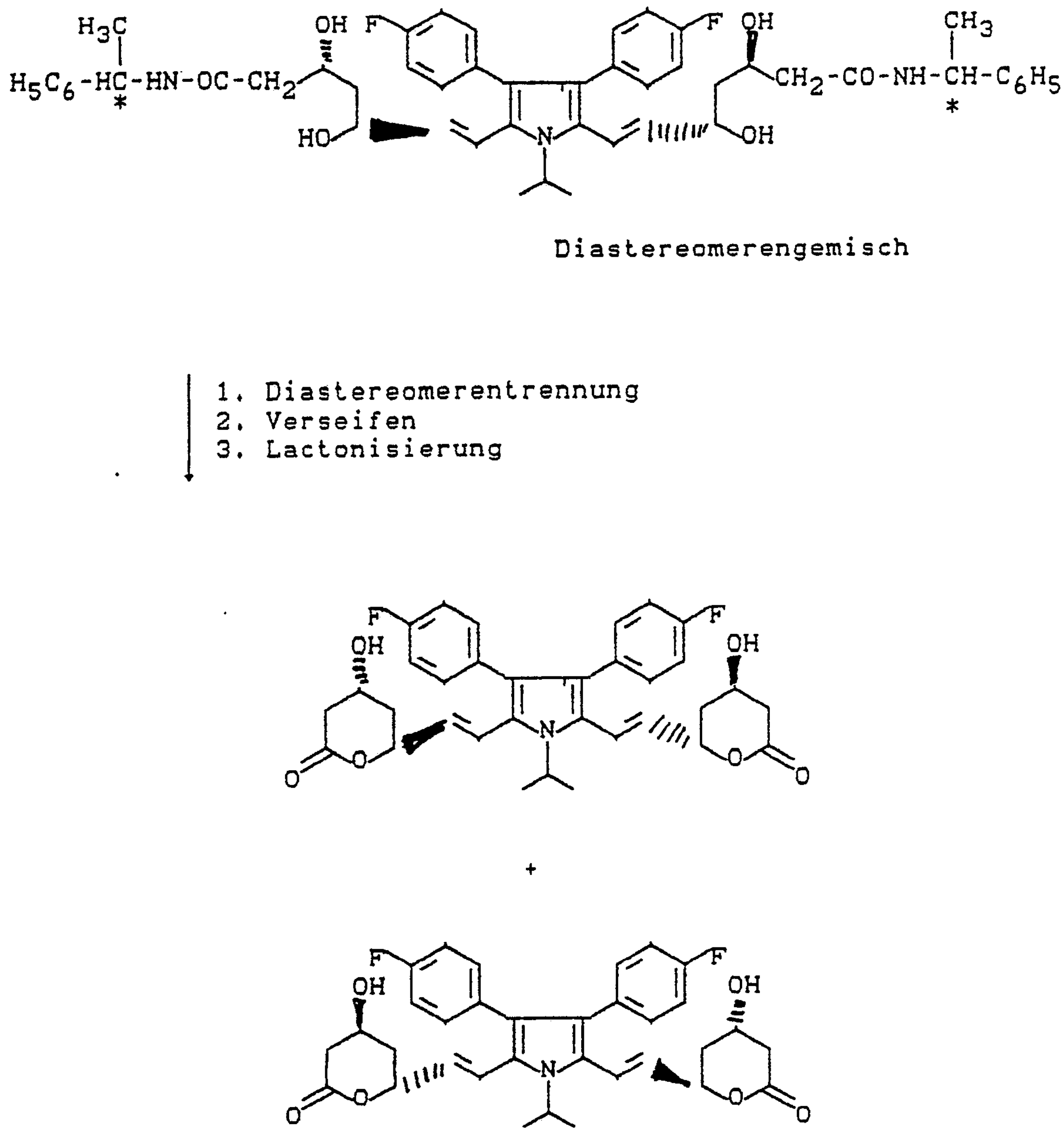


Diastereomerengemisch

+

Die als Ausgangsstoffe eingesetzten Ketone (VIII) sind neu.

Es wurde ein Verfahren zur Herstellung der erfindungsgemäßen Ketone der allgemeinen Formel (VIII)

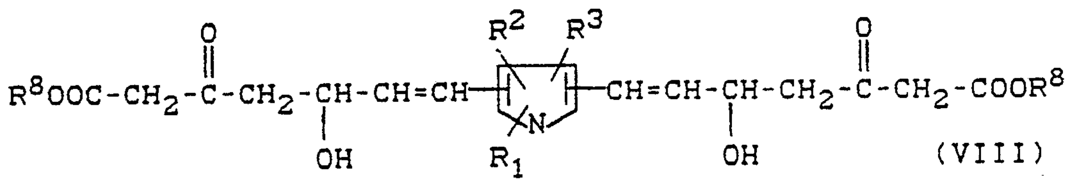

(VIII)

in welcher

$R^1$, $R^2$, $R^3$ und $R^8$ die oben genannte Bedeutung haben,

gefunden, das dadurch gekennzeichnet ist, daß man Aldehyde der allgemeinen Formel (IX)

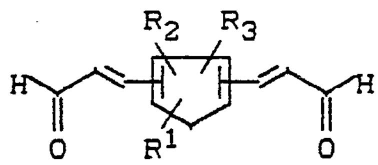

(IX)

in welcher

$R^1$, $R^2$ und $R^3$ die oben angegebene Bedeutung haben,

in inerten Lösemittelen mit Acetessigester der allgemeinen Formel (X)

$H_3C-\overset{\overset{O}{||}}{C}-CH_2-COOR^8$ (X)

in welcher

$R^8$ die oben angegebene Bedeutung hat,

in Anwesenheit von Basen umsetzt.

Das erfindungsgemäße Verfahren kann beispielsweise durch folgendes Formelschema erläutert werden:

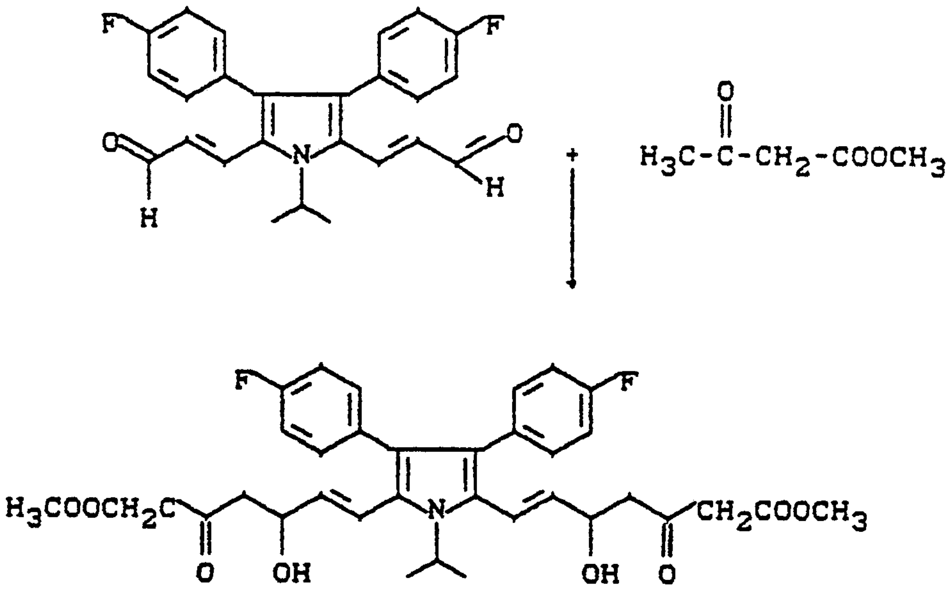

Als Basen kommen hierbei übliche stark basischen Verbindungen in Frage. Die Reaktion wird auch in Gegenwart von Zinksalzen durchgeführt. Hierzu gehören bevorzugt lithiumorganische Verbindungen wie beispielsweise n-Butyllithium, sec.Butyllithium, tert.Butyllithium oder Phenyllithium, oder Amide wie beispielsweise Lithiumdiisopropylamid, Natriumamid oder Kaliumamid, oder Lithiumhexamethyldisilylamid, oder Alkalihydride wie Natriumhydrid oder Kaliumhydrid. Ebenso ist es möglich, Gemische der genannten Basen einzusetzen. Besonders bevorzugt sind n-Butyllithium, Natriumhydrid oder deren Gemisch in Gegenwart von Zinkbromid.

Als Lösemittel eignen sich hierbei die üblichen organischen Lösemittel, die sich unter den Reaktionsbedingungen nicht verändern. Hierzu gehören bevorzugt Ether wie Diethylether, Tetrahydrofuran, Dioxan oder Dimethoxyethan, oder Kohlenwasserstoffe wie Benzol, Toluol, Xylol, Cyclohexan, Hexan oder Erölfraktionen. Ebenso ist es möglich, Gemische der genannten Lösemittel einzusetzen. Besonders bevorzugt werden Ether wie Diethylether oder Tetrahydrofuran verwendet.

Die Reaktion wird im allgemeinen in einem Temperaturbereich von -80 °C bis +50 °C, bevorzugt von -20 °C bis +30 °C durchgeführt.

Das Verfahren wird im allgemeinen bei Normaldruck durchgeführt, es ist aber auch möglich das Verfahren bei Unterdruck oder bei Überdruck durchzuführen, z.B. in einem Bereich von 0,5 bis 5 bar.

Bei der Durchführung des Verfahrens wird der Acetessigester im allgemeinen in einer Menge von 1 bis 2, bevorzugt von 1 bis 1,5 mol, bezogen auf 1 mol des Aldehyds eingesetzt.

Die als Ausgangsstoffe eingesetzten Acetessigester der Formel (X) sind bekannt oder können nach bekannten Methoden hergestellt werden [Beilstein's Handbuch der organischen Chemie III, 632; 438].

Als Acetessigester für das erfindungsgemäße Verfahren seien beispielsweise genannt:

Acetessigsäuremethylester, Acetessigsäureethylester, Acetessigsäurepropylester, Acetessigsäureisopropylester.

Die als Ausgangsstoffe eingesetzten Aldehyde der allgemeinen Formel (IX) sind neu.

Es wurde außerdem ein Verfahren zur Herstellung der Aldehyde der allgemeinen Formel (IX)

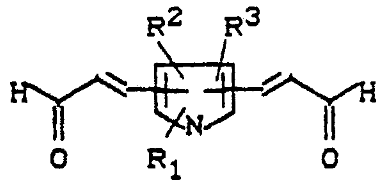

in welcher
$R^1$, $R^2$ und $R^3$ die oben angegebene Bedeutung haben,
gefunden, das dadurch gekennzeichnet ist, daß man
Pyrrole der allgemeinen Formel (XI)

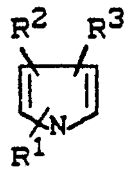

(XI),

in welcher
$R^1$, $R^2$ und $R^3$ die oben angegebene Bedeutung haben, in inerten Lösemitteln in Anwesenheit von Hilfstoffen mit N,N-Dialkylaminoacrolein der Formel (XII)

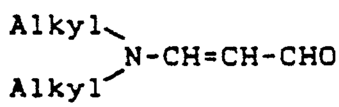

(XII)

wobei
Alkyl für einen geradkettigen oder verzweigten Kohlenstoffrest mit 1 bis 6 Kohlenstoffatomen steht,
umsetzt.

Das erfindungsgemäße Verfahren kann beispielsweise durch folgendes Formelschema verdeutlicht werden:

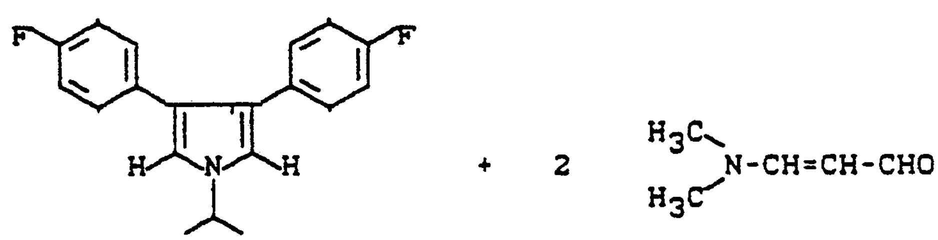

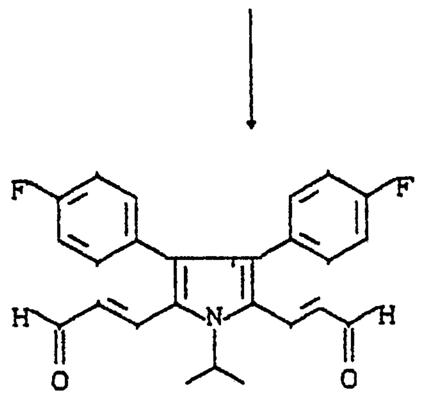

Als Lösemittel eignen sich hierbei die üblichen organischen Lösemittel, die unter den Reaktionsbedingungen stabil sind. Hierzu gehören bevorzugt Kohlenwasserstoffe wie Benzol, Toluol, Xylol, Hexan, Erdölfraktionen, Chlorbenzol oder o-Dichlorbenzol, oder Ether wie Diethylether, Dioxan oder Tetrahydrofuran, oder Chlorkohlenwasserstoffe wie Methylenchlorid, Chloroform oder Tetrachlorkohlenstoff, oder Acetonitril. Ebenso ist es möglich, Gemische der genannten Lösemittel einzusetzen. Besonders bevorzugt wird wasserfreies Acetonitril oder Chloroform verwendet.

Als Hilfsstoffe werden im allgemeinen Säurechloride verwendet. Bevorzugt wird Phosphoroxychlorid oder Phosgen, besonders bevorzugt Phosphoroxychlorid eingesetzt.

Die Reaktion wird in einem Temperaturbereich von -20° C bis +150° C, bevorzugt von 0° C bis 100° C durchgeführt.

Das Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung des Verfahrens wird das Dimethylaminoacrolein im allgemeinen in einer Menge von 1 bis 6, bevorzugt von 1,2 bis 3 mol, bezogen auf 1 mol des Pyrrols eingesetzt.

Die als Ausgangsstoffe eingesetzten Pyrrole der allgemeinen Formel (XI) sind bekannt oder können nach bekannten Methoden hergestellt werden [A. Glossauer "Die Chemie der Pyrrole", Springer Verlag Berlin, 1974].

Die erfindungsgemäßen disubstutierten Pyrrole können in Arzneimitteln zur therapeutischen Behandlung beim Mensch und beim Tier verwendet werden. Bevorzugt können sie als Inhibitoren der 3-Hydroxy-3-methylglutaryl-Coenzym A (HMG-CoA) Reduktase und Inhibitoren der Cholesterolbiosynthese verwendet werden. Sie können deshalb zur Behandlung von Hyperlipoproteinämie, Lipoproteinämie oder Arteriosklerose eingesetzt werden, Die erfindungsgemäßen Wirkstoffe bewirken außerdem eine Senkung des Cholesteringehaltes im Blut.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung verhanden sein, d.h. in Mengen, die ausreichend sind, um den angegebenen Dosierungspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benzutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B.: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin- Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffe, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeit punkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Ausgangsverbindungen und Herstellungsbeispiele

Beispiel 1

1,2-Bis-(4-fluorphenyl)-acrylnitril

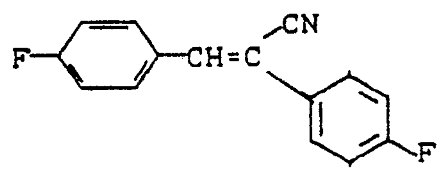

Zu einer 50 °C warmen Lösung von 137,7 g (1,11 mol) 4-Fluorbenzaldehyd und 150 g (1,11 mol) 4-Fluorbenzylcyanid in 900 ml Ethanol tropft man eine Lösung von 2,3 g Natrium in 35 ml Ethanol. Dabei fällt ein voluminöser farbloser Niederschlag aus der nach 1 h Nachrühren ohne Heizung abgesaugt wird. Man wäscht mit Wasser nach und trocknet über Phosphorpentoxid im Hochvakuum

Ausbeute: 258 g (96 % der Theorie)

Schmp.: 164 °C

Beispiel 2

2-Ethoxycarbonyl-3,5-bis-(4-fluorphenyl)-pyrrol

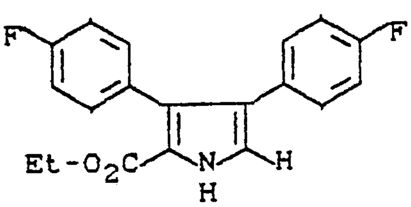

Zu einer Suspension von 5,1 g (0,17 mol) 80 %igem Natriumhydrid in 125 ml wasserfreiem Tetrahydrofuran tropft man bei -15 °C eine warme Lösung von 18 g (0,16 mol) Isocyanessigsäureethylester und 29,4 g

(0,12 mol) der Verbindung aus Beispiel 1 in 15 ml Dimethylsulfoxid und 125 ml Tetrahydrofuran, rührt eine weitere Stunde bei -15 °C und schließlich 30 min bei Raumtemperatur. Nun wird vorsichtig Wasser zugetropft, die wäßrige Phase abgetrennt, dreimal mit Essigester extrahiert und die vereinigten organischen Phasen mit gesättigter Natriumchlorid-Lösung gewaschen. Nach Trocknen über Natriumsulfat engt man zu einem braunen Öl ein (48 g), das mit Toluol über 200 g Kieselgel filtriert wird. Aus Ether kristallisieren 16,2 g (41 % der Theorie), fast farblose Kristalle vom Schmelzpunkt 138 °C.

Beispiel 3

2-Ethoxycarbonyl-3,4-bis(4-fluorphenyl)-1-isopropyl-pyrrol

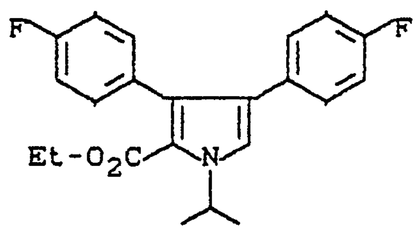

Zu 5,14 g (45,8 mmol) Kaliumtertiärbutoxid in 40 ml wasserfreiem Tetrahydrofuran tropft man bei 0 °C 10 g (30,6 mmol) der Verbindung aus Beispiel 2 in 50 ml Tetrahydrofuran, gefolgt von 15,6 g (91,8 mmol) Isopropyliodid. Man kocht 18 h unter Rückfluß und gießt dann auf 300 ml Eiswasser. Nach Zugabe von 300 ml Wasser, wird dreimal mit je 150 ml Essigester extrahiert, die organische Phase mit gesättigter Natriumchlorid-Lösung gewaschen, über Natriumsulfat getrocknet und eingeengt (13 g braunes Öl). Nach Filtrieren über 100 g Kieselgel (230-400 mesh) mit Petrolether-Dichlormethan (3:1) erhält man 9,2 g (81 % der Theorie) schwach gefärbtes Öl, das im Kühlschrank erstarrt.

Schmp.: 49 °C

$^1$H-NMR ($CDCl_3$): $\delta$ = 0,95 (t, 3H, $CH_2$-$\underline{CH_3}$), 1,53 (d, 6H, CH-($\underline{CH_3}$)$_2$); 4,05 (q, 2H, $\underline{CH_2}$-$CH_3$); 5,43 (sept, 1H, $\underline{CH}$($CH_3$)$_2$; 6,8 -7,2 (m, 9H, 5H + Aromaten-H).

Beispiel 4

3,4-Bis-(4-fluorphenyl)1-isopropyl-pyrrol-2-carbonsäure

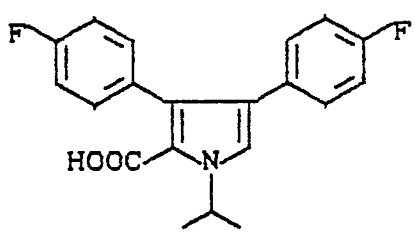

6,8 g (18,4 mol) der Verbindung aus Beispiel 3 werden in 30 ml Ethanol und 3,4 ml 6 N Natronlauge 18 h am Rückfluß gekocht. Dann wird mit 1 N Salzsäure angesäuert und der ausgefallene Niederschlag abgesaugt. Man löst den Niederschlag wieder in Essigester, extrahiert mit 1 n Salzsäure und gesättigter Natriumchlorid-Lösung, trocknet über Natriumsulfat und engt zur Trockene ein. Umkristallisation aus Ether/Petrolether ergibt 4,35 g (69 % d.Th.) farblose Kristalle vom Schmelzpunkt 212 °C.

Beispiel 5

3,4-Bis-(4-Fluorphenyl)-1-isopropyl-pyrrol

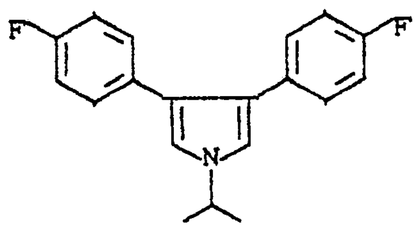

A:

4,6 g (13,5 mmol) der Verbindung aus Beispiel 4 werden in 15 ml Essigsäure 30 min. am Rückfluß gekocht. Man zieht die Essigsäure im Vakuum ab, löst den Rückstand in Dichlormethan, wäscht mit Natriumhydrogencarbonat-Lösung und Natriumchlorid-Lösung und trocknet über Natriumsulfat. Nach dem Einengen bleiben 3,9 g (97 % der Theorie) farblose Kristalle vom Schmelzpunkt 105° C.

B:

Unter Eiskühlung tropft man zu 2,22 g (19,8 mmol) Kaliumtertiärbutoxid in 15 ml Tetrahydrofuran eine Lösung von 3,37 g (13,2 mmol) der Verbindung aus Beispiel 11 in 15 ml Tetrahydrofuran. Bei Raumtemperatur tropft man dann (39,6 mmol) Isopropyliodid zu und kocht 3 h am Rückfluß.

Nach Zugabe von 50 ml Wasser wird dreimal mit Methylenchlorid extrahiert, die vereinigten organischen Phasen getrocknet und zu einem gelben Öl eingeengt. Filtration über 50 g Kieselgel mit Petrolether/Methylenchlorid (10:1) ergibt 2,13 g (54 %) farbliche Kristalle vom Schmp. 104° C (im DC identisch mit der nach Verfahren A erhaltenen Probe).

Beispiel 6

3,4-Bis-(4-fluorphenyl)2-5-bis-(2-formyl-ethenyl)-1-isopropyl-pyrrol

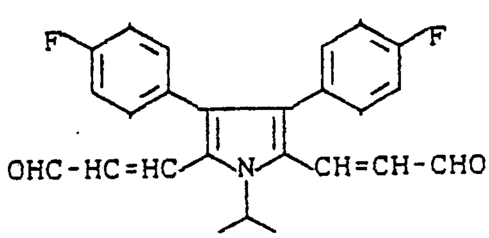

Zu 3,82 ml (41,9 mmol) Phosphoroxidchlorid in 20 ml wasserfreiem Acetonitril tropft man bei - 5° C eine Lösung aus 4,3 g (39,1 mmol) 90 %igem Dimethylaminoacrolein in 10 ml Acetonitril und gibt dann portionsweise 2,1 g (7,1 mmol) der Verbindung aus Beispiel 5 zu. Es wird 18 h am Rückfluß gekocht, dann auf eine kalte Emulsion von 200 ml Toluol und 200 ml Wasser, worin 13 g Natriumhydroxid gelöst sind, gegeben und 1,5 h bei Raumtemperatur kräftig durchgerührt. Man saugt über Kieselgur ab, trocknet die organische Phase über Natriumsulfat und engt zu einem schwarzen Öl ein (3,2 g). Säulenchromatographie an 60 g Kieselgel (230 -400 mesh) mit je 300 ml Toluol, Toluol/Essigester (10:1), (5:1) und (3:1) ergibt zwei

Fraktionen: Zuerst eluiert man 0,88 g (36 %) 3,4-Bis-(4-fluorphenyl)-2-(2-formyl-ethenyl)-1-isopropyl-pyrrol als schwach gefärbte Kristalle vom Schmelzpunkt 153° C, dann das Produkt, 1,4 g gelber Schaum, der aus Ether/Petrolether umkristallisiert 1,12 g (39 %) orangefarbene Kristalle vom Schmelzpunkt 207° C ergibt.

Beispiel 7

3,4-Bis-(4-fluorphenyl)-2,5-bis-(3-hydroxy-6-methoxycarbonyl-5-oxo-hex-1-enyl)-1-isopropyl-pyrrol

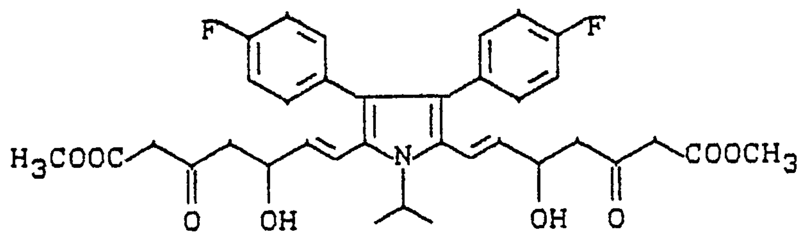

Unter Argon gibt man zu einer Suspension von 107 mg (3,56 mmol) 80 %igem Natriumhydrid in 10 ml wasserfreiem Tetrahydrofuran bei -5° C langsam 0,35 ml (3,25 mmol) Acetessigsäuremethylester und dann bei ca. 0° C langsam 2,72 ml (4,43 mmol) einer 15 %igen Lösung von Butyllithium in Hexan. Nach 15 min. Rühren bei 0° C wird eine Lösung von 0,6 g (1,48 ml) der Verbindung aus Beispiel 6 in 5 ml Tetrahydrofuran zugetropft und weitere 15 min bei derselben Temperatur gerührt. Schließlich tropft man vorsichtig 0,58 g (9,67 mmol) Essigsäure und dann 50 ml Wasser zu, extrahiert dreimal mit Essigester und trocknet die organische Phase über Natriumsulfat und Natriumcarbonat. Nach dem Abziehen des Lösemittels bleiben 0,94 g rötlicher Schaum vom $R_f$-Wert: 0,2 mit Toluol/Essigester (1:1). Das Rohprodukt wird weiterverarbeitet.

Beispiel 8

3,4-Bis-(4-fluorphenyl)-2,5-bis(3,5-dihydroxy-6-methoxycarbonyl-hex-1-enyl)-1-isopropyl-pyrrol

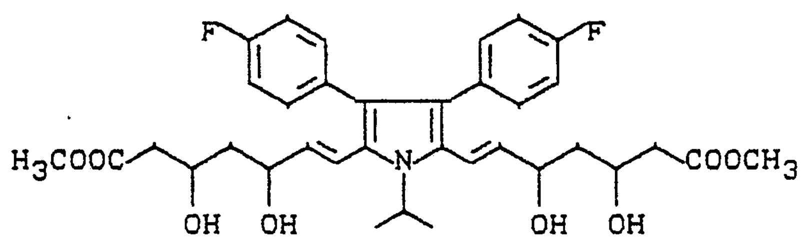

Unter Argon tropft man bei Raumtemperatur zu einer Lösung von 0,94 g (1,48 mol) der Verbindung aus Beispiel 7 in 10 ml wasserfreiem Tetrahydrofuran 3,56 ml einer 1 M Lösung von Triethylboran in Tetrahydrofuran und leitet 5 min lang Luft durch die Lösung. Nach Abkühlen auf -78° C werden 138 mg (3,64 mol) Natriumborhydrid zugegeben, dann langsam 1,96 ml wasserfreies Methanol zugetropft, so daß die Temperatur unter -65° C bleibt, 15 min bei ca. -75° C und 15 min. bei -30° C gerührt, bevor bei 0° C 9,9 ml 30 %iges Wasserstoffperoxid in 30 ml Wasser zugetropft werden. Nach Erwärmen auf Raumtemperatur extrahiert man dreimal mit 30 ml Essigester, wäscht die vereinigten organischen Phasen mit gesättigter Natriumchlorid-Lösung, trocknet über Natriumsulfat und Natriumcarbonat und engt zu einem rotbraunem Schaum (850 mg) ein. Nach zweimaliger Säulenchromatographie an der 30fachen Menge Kieselgel (230-400 mesh) zuerst mit Toluol/Essigester (1:1) und Essigester, zweitens mit Dichlormethan/Essigester (1:1) und (1:3) erhält man 102 mg (11 % der Theorie) fast farblosen amorphen Feststoff.

$R_f$ = 0,3, Essigester
$^1$H-NMR ($CDCl_3$): δ = 1,3 - 1,6 (m, 10H, CH-($CH_3$)$_2$ + CH(OH)-$CH_2$-CH(OH); 2,45 (m, 4H, $CH_2$-$COOCH_3$); 3,18 (d, 2H, OH); 3,62 (d, 2H, OH); 3,7 (s, 6H, $COOCH_3$); 4,13 (m, 2H, CH-OH); 4,38 (m, 2H, CH-OH); 4,7 (m, 2H, CH($CH_3$)$_2$); 5,32 (dd, 2H, Olefin-H); 6,1 (d,2H, Olefin-H); 6,85 (m, 4H, 3′-H); 7,0 (m, 4H, 2′-H).

Beispiel 9

3,4-Bis-(4-fluorphenyl)-1-isopropyl-2,5-pyrrol-bis-(3,5-dihydroxy-hept-6-en-säure-di-Natriumsalz

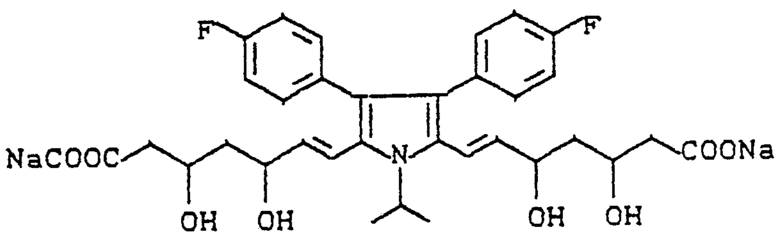

86 mg (0,13 mmol) der Verbindung aus Beispiel 8 werden in 2 ml Tetrahydrofuran mit 0,25 ml 1 M wäßriger Natriumhydroxid-Lösung 1,5 h bei Raumtemperatur gerührt. Es wird einrotiert und über Phosphorpentoxid im Hochvakuum getrocknet.
Ausbeute: 72 mg (81 % der Theorie) amorpher gelblicher Feststoff
Zers. ab 200° C
$^1$H-NMR ($CD_3OD$): δ = 1,25 - 1,6 (m, 10H, CH-($CH_3$)$_2$ + CH(OH)$CH_2$-CH(OH)); 2,25 (m, 4H, $CH_2$-$COOCH_3$); 3,85 (m, 2H, CH-OH); 4,25 (m, 2H, CH-OH); 5,35 (m, 2H, Olefin-H); 6,6 (d, 2H, Olefin-H); 6,8 - 7.1 (m, 8H, Aromaten-H).

Beispiel 10

4-Cyano-3,4-bis(4-fluorphenyl)-3H-4,5-dihydropyrrol-2-carbonsäure-Natriumsalz

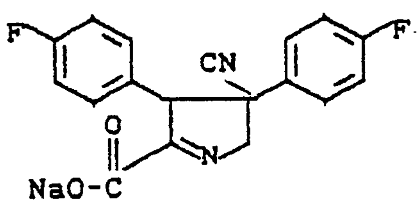

Zu einer Suspension von 17,7 g (0,59 mol) 80 %igem Natriumhydrid in 450 ml wasserfreiem Tetrahydrofuran tropft man bei -15° C eine warme Lösung von 62 g (0,55 mol) Isocyanessigsäureethylester und 101 g (0,42 mol) der Verbindung aus Beispiel 1 in 50 ml Dimethylsulfoxid und 450 ml Tetrahydrofuran und rührt eine Stunde bei -15° C. Bei dieser Temperatur werden 500 ml Wasser vorsichtig zugetropft, dann läßt man unter Rühren auf Raumtemperatur erwärmen. Man extrahiert viermal mit Essigester und verwirft die org. Phasen. Aus der Wasserphase fällt ein voluminöser farblicher Niederschlag, der abgesaugt und im Vakuum über Phosphorpentoxid getrocknet wird.

Ausbeute 95 g (62 %). Durch Umkristallisieren aus Wasser erhält man farblose Kristalle, die sich bei 193-194° C zersetzen, dann fest werden und bei 252-253° C erneut unter Zersetzung schmelzen.

| $C_{18}H_{11}F_2NaNO_3 \bullet H_2O$ (366.3) | | | | | |
|---|---|---|---|---|---|
| | C | H | F | Na | N |
| Ber.: | 59,0 | 3,6 | 10,4 | 6,3 | 7,6 |
| Gef.: | 58,9 | 3,3 | 10,5 | 6,0 | 7,5 |

$^1$H-NMR ($D_6$-DMSO): $\delta$ = 4,5 (m, 2H, $CH_2$) 4,7 (s, 1H, 3-H) 7,1 - 7,9 (m, 8H, Ar-H)

Beispiel 11

3,4-Bis-(4-fluorphenyl)-pyrrol

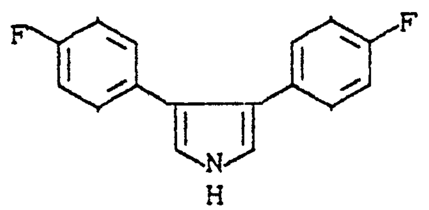

1,63 g (4,45 mmol) der Verbindung aus Beispiel 10 und 3 g Ethanolamin werden 3 h auf 100° C erhitzt. Die Lösung wird mit 50 g Eiswasser versetzt und dreimal mit Essigester extrahiert. Die vereinigten org. Phasen werden mit 1N Salzsäure, ges. Bicarbonatlösung und ges. Kochsalzmischung gewaschen, über Natriumsulfat getrocknet und eingeengt. Den Rückstand (1,7 g braunes Öl) filtriert man über 50 g Kieselgel mit Petrolether-Methylenchlorid (2:1).
Ausbeute: 0,77 g (68 % der Theorie) farbliche Kristalle vom Schmp. 142° C.

Anwendungsbeispiel

Beispiel 12

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Cholestyramin/kg Futter zugesetzt war, behandelt. Nach Dekapitation wurde den Tieren die Leber entnommen und auf Eis gegeben. Die Lebern wurden zerkleinert und in einem Homogenisator 3 mal in 3 Volumen 0,1 m Saccharose, 0,05 m KCl, 0,04 m $K_xH_y$ Phosphat (Mischung aus $K_2HPO_4$ und $KH_2PO_4$ mit pH 7,2), 0,03 m Ethylendiamintetraessigsäure, 0,002 m Dithiothreit (SPE)-Puffer (Saccharose-Phosphat-Ethylendiamintetraessigsäure-Puffer) pH 7,2 homögenisiert. Anschließend wurde 15 Minuten zentrifugiert und das Sediment verworfen. Der Überstand wurde 75 Minuten sedimentiert. Das Pellet wird in 1/4 Volumen SPE-Puffer aufgenommen, nochmals homogenisiert und anschließend erneut 60 Minuten zentrifugiert. Das Pellet wird mit der 5-fachen Menge ihres Volumens SPE-Puffer aufgenommen, homogenisiert und bei -78° C eingefroren und gelagert (= Enzymlösung).

Zur Testung wurden die Testverbindungen (bzw. Mevinolin als Referenzsubstanz) in Dimethylformamid unter Zugabe von 5 Vol-% 1 n NaOH gelöst und mit 10 µl in verschiedenen Konzentrationen in den Enzymtest eingesetzt. Der Test wurde nach 20 Minuten Vorinkubation der Verbindungen mit dem Enzym bei 37° C gestartet. Der Testansatz betrug 0,380 ml und enthielt 4 µMol Glucose-6-Phosphat, 1,1 mg rinderserumalbumin, 2,1 µMol Dithiothreit, 0,35 µMol NADP, 1 Einheit Glucose-6-Phosphatdehydrogenase 35, µMol $K_xH_y$-Phosphat PH 7,2, 20 µl Enzympräparation und 56 nMol 3xHydroxy-3-methyl-glutaryl Coenzym A (Glutaryl-3-$^{14}$C) 100 000 dpm.

Man inkubierte 60 Minuten bei 37° C und stoppte die Reaktion durch Zusatz von 300 µl 0,24 m HCl ab. Nach einer Nachinkubation von 60 Minuten bei 37° C wurde der Ansatz zentrifugiert und 600 µl des Überstandes auf eine 0,7 x 4 cm mit 5-Chlorid Anionenaustauscher mit einer Korngröße 100-200 mesh

(Anionenaustauscher) gefüllte Säule aufgetragen. Es wurde mit 2 ml dest. Wasser nachgewaschen und durchlauf plus Waschwasser mit 3 ml einer Scintillationsflüssigkeit versetzt und im Scintillationszähler gezählt. $IC_{50}$-Werte wurden durch Auftrag der przentualen Hemmung gegen die Konzentration der Verbindung im Test durch Intrapolation bestimmt. Zur Bestimmung der relativen inhibitorischen Potenz wurde der $IC_{50}$-Wert der Referenzsubstanz Mevinolin als 1 gesetzt und mit dem simultan bestimmten $IC_{50}$-Wert der Testverbindung verglichen.

Die erfindungsgemäßen Wirkstoffe zeigen eine höhere Wirkung als Mevinolin.

| Beispiel-Nr.: | relative Aktivität (Mevinolin = 1) |
|---|---|
| 9 | 40 |
| [≙ Beispiel 9 ist 40 x besser als Mevinolin] | |

Beispiel 13

Die subchronische Wirkung der erfindungsgemäßen Verbindungen auf die Blutcholesterinwerte von Hunden wurde in mehrwöchigen Fütterungsexperimenten geprüft. Dazu wurde die zu untersuchende Substanz über einen mehrwöchigen Zeitraum einmal täglich in einer Kapsel gesunden Beagle Hunden zusammen mit dem Futter p.o. gegeben. Dem Futter war außerdem während der gesamten Versuchsperiode, d.h. vor, während und nach der Applikationsperiode der zu untersuchenden Substanz, Colestyramin (4 g/100 g Futter) als Gallensäuresequestrant beigemischt. Zweimal wöchentlich wurde den Hunden venöses Blut entnommen und das Serumcholesterin enzymatisch bestimmt. Die Serumcholesterinwerte während der Applikationsperiode wurden mit den Serumcholesterinwerten vor der Applikationsperiode (Kontrolle) verglichen.

## Ansprüche

1. Disubstituierte Pyrrole der Formel

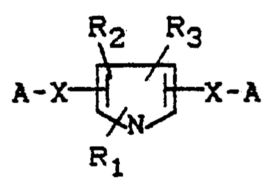

(I)

in welcher

$R^1$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,

worin

$R^4$, $R^5$ - gleich oder verschieden sind und Alkyl, Aryl, Aralkyl, Acyl, Alkylsulfonyl oder Arylsulfonyl bedeuten,

oder

- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,

worin

$R^4$ und $R^5$ die oben angegebene Bedeutung haben,

$R^2$ - Cycloalkyl bedeutet, oder

- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,

worin

$R^4$, $R^5$ - die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,

$R^3$ - Wasserstoff bedeutet, oder

- Cycloalkyl bedeutet, oder

- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,

worin

$R^4$, $R^5$ die oben angegebene Bedeutung haben,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein können,

oder

- Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,

worin

$R^4$ und $R^5$ die oben angegebene Bedeutung haben,

oder

- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,

worin

$R^4$ und $R^5$ die oben angegebene Bedeutung haben,

X - eine Gruppe der Formel $-CH_2-CH_2-$ oder $-CH=CH-$ bedeutet,

und

A - eine Gruppe der Formel

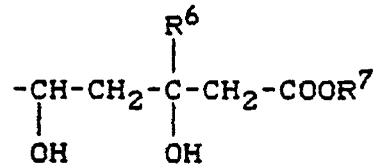 oder 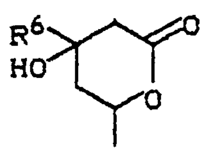 bedeutet,

worin

$R^6$ - für Wasserstoff oder Alkyl steht,

und

$R^7$ - Wasserstoff bedeutet oder

- für einen Alkyl, Aryl oder einen Aralkylrest oder

- für ein Kation steht.

2. Disubstituierte Pyrrole nach Anspruch 1, worin

$R^1$ - Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder

- Phenyl oder Naphthyl bedeutet, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^4R^5$,
wobei
$R^4$ und $R^5$ gleich oder verschieden sind und Niederalkyl, Phenyl, Benzyl, Acetyl, Benzoyl, Phenylsulfonyl oder Niederalkylsulfonyl bedeuten,
$R^2$ - Cyclopropyl, Cyclopentyl oder Cyclohexyl bedeutet, oder
- Niederalkyl bedeutet, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^4R^5$,
worin
$R^4$ und $R^5$ die oben angegebene Bedeutung haben, oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,
$R^3$ - Wasserstoff, oder
- Cyclopropyl, Cyclopentyl oder Cyclohexyl bedeutet, oder
- Niederalkyl bedeutet, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, oder durch eine Gruppe der Formel $-NR^4R^5$,
worin
$R^4$ und $R^5$ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl-und Arylreste bis zu 2-fach gleich oder verschieden durch Fluor, Chlor, Brom, Niederalkyl, Niederalkoxy, Trifluormethyl, oder Trifluormethoxy substituiert sein können,
- Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
- Phenyl oder Naphthyl bedeutet, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^4R^5$,
wobei
$R^4$ und $R^5$ die oben angegebene Bedeutung haben,
X - eine Gruppe der Formel -CH=CH- bedeutet
und
A - eine Gruppe der Formel

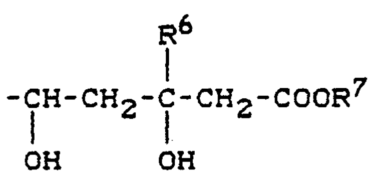 oder 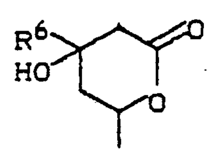 bedeutet,

worin
$R^6$ - Wasserstoff oder Niederalkyl bedeutet,
und
$R^7$ - einen $C_1$ bis $C_6$ Alkylrest, ein $C_6$ bis $C_{12}$ Arylrest oder ein $C_7$ bis $C_{10}$ Aralkylrest bedeutet, oder
- ein physiologisch verträgliches Kation bedeutet.

3. Disubstituierte Pyrrole nach den Ansprüchen 1 und 2,
worin
$R^1$ - Pyridyl, Pyrimidyl, Chinolyl oder Isochinolyl bedeutet, das durch Fluor, Chlor, Methyl, Methoxy oder

Trifluormethyl substituiert sein kann, oder

- Phenyl bedeutet, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,

$R^2$ - Cyclopropyl, Cyclopentyl oder Cyclohexyl bedeutet,

oder

- Methyl, Ethyl, Propyl, Isopropyl, Butyl, sec.Butyl oder tert.Butyl bedeutet, das substituiert sein kann durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxy carbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,

$R^3$ - Wasserstoff, Cyclopropyl, Cyclopentyl oder Cyclohexyl bedeutet, oder

- Methyl, Ethyl, Propyl, Isopropyl, Butyl, Isobutyl, tert.Butyl, Pentyl, Isopentyl, Hexyl oder Isohexyl bedeutet, das substituiert sein kann durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe $-NH^4R^5$,

wobei

$R^4$ und $R^5$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,

oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder

- Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl bedeutet, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder

- Phenyl bedeutet, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^4R^5$ substituiert sein kann,

wobei

$R^4$ und $R^5$ die oben angegebene Bedeutung haben,

X - eine Gruppe der Formel $-CH=CH-$ bedeutet

und

A - eine Gruppe der Formel

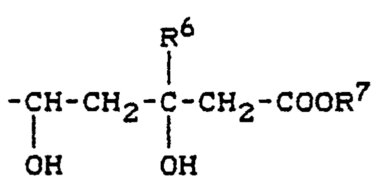 oder 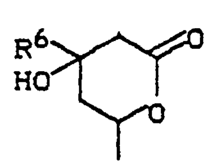 bedeutet,

worin
$R^6$ - Wasserstoff, Methyl, Ethyl, Propyl, Isopropyl, Butyl, Isobutyl oder tert.Butyl bedeutet
und
$R^7$ - Wasserstoff, Methyl, Ethyl, Propyl, Isopropyl, Butyl, Isobutyl, tert.Butyl oder Benzyl bedeutet, oder
- ein Natrium-, Kalium-, Calcium- oder Magnesium- oder Ammoniumion bedeutet.

4. Disubstituierte Pyrrole nach Anspruch 1
zur therapeutischen Behandlung.

5. Verfahren zur Herstellung von disubstituierten Pyrrolen der Formel

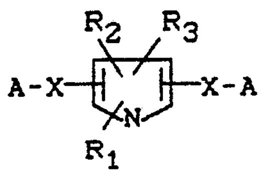

(I)

in welcher
$R^1$ - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann
worin
$R^4$, $R^5$ - gleich oder verschieden sind und Alkyl, Aryl, Aralkyl, Acyl, Alkylsulfonyl oder Arylsulfonyl bedeuten,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,
worin
$R^4$ und $R^5$ die oben angegebene Bedeutung haben,
$R^2$ - Cycloalkyl bedeutet, oder
- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,
worin
$R^4$, $R^5$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
$R^3$ - Wasserstoff bedeutet, oder
- Cycloalkyl bedeutet, oder
- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^4R^5$,
worin
$R^4$, $R^5$ die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio oder Alkylsulfonyl substituiert sein können,
oder
- Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^4R^5$ substituiert sein kann,
worin
$R^4$ und $R^5$ die oben angegebene Bedeutung haben,

oder

- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^4R^5$,

worin

$R^4$ und $R^5$ die oben angegebene Bedeutung haben,

X - eine Gruppe der Formel $-CH_2-CH_2-$ oder $-CH=CH-$bedeutet,

und

A - eine Gruppe der Formel

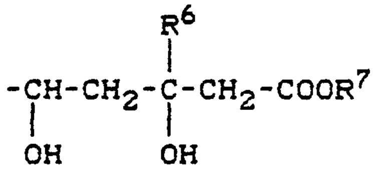

oder

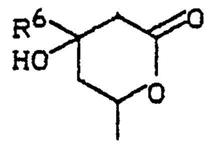

bedeutet,

worin

$R^6$ - für Wasserstoff oder Alkyl steht,

und

$R^7$ - Wasserstoff bedeutet oder

- für einen Alkyl, Aryl oder einen Aralkylrest oder

- für ein Kation steht,

dadurch gekennzeichnet, daß man

Ketone der allgemeinen Formel (VIII)

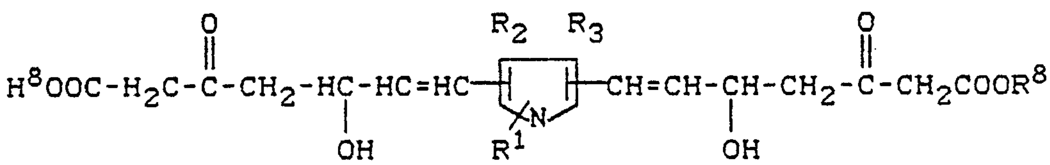

in welcher

$R^1$, $R^2$ und $R^3$ die oben angebebene Bedeutung haben,

und

$R^8$-für Alkyl steht,

reduziert,

im Fall der Herstellung der Säuren die Ester verseift,

im Fall der Herstellung der Lactone die Carbonsäuren cyclisiert,

im Fall der Herstellungder Salze entweder die Ester oder die Lactone verseift,

im Fall der Herstellung der Ethylenverbindungen ($X = -CH_2-CH_2-$) die Ethenverbindungen ($X = -CH=CH-$) nach üblichen Methoden hydriert,

und gegebenenfalls Isomeren trennt.

6. Verfahren nach Anspruch 5, dadurch gekennzeichnet, daß es im Temperaturbereich von - 90 bis + 30 °C durchgeführt wird.

7. Ketone der Formel

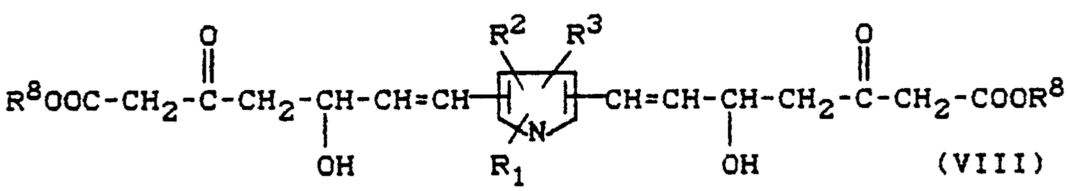

(VIII)

in welcher

$R^1$, $R^2$ und $R^3$ die in Anspruch 1 angegebene Bedeutung haben,
und
$R^8$ für Alkyl steht. 8. Verfahren zur Herstellung von Ketonen der Formel

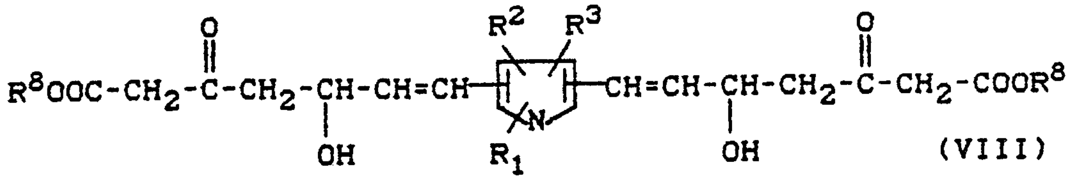

(VIII)

in welcher
$R^1$, $R^2$ und $R^3$ die in Anspruch 5 angegebene Bedeutung haben,
und
$R^8$ für Alkyl steht,
dadurch gekennzeichnet, daß man
Aldehyde der allgemeinen Formel (IX)

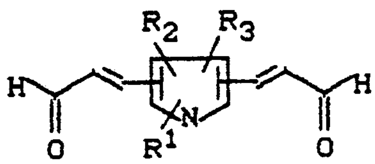

(IX)

in welcher
$R^1$, $R^2$ und $R^3$ die oben angegebene Bedeutung haben,
in inerten Lösemitteln mit Acetessigester der allgemeinen Formel (X)

$$H_3C-\overset{\overset{\displaystyle O}{\|}}{C}-CH_2-COOR^8 \qquad (X)$$

in welcher
$R^8$ die oben angegebene Bedeutung hat,
in Anwesenheit von Basen umsetzt.

9. Aldehyde der Formel

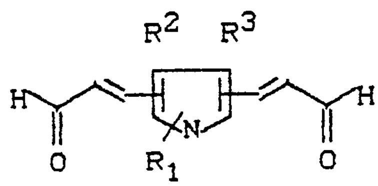

in welcher
$R^1$, $R^2$ und $R^3$ die in Anspruch 1 angegebene Bedeutung haben.

10. Verfahren zur Herstellung von Aldehyden der Formel

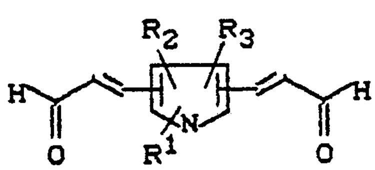

(IX)

in welcher
$R^1$, $R^2$ und $R^3$ die in Anspruch 5 angegebene Bedeutung haben,
dadurch gekennzeichnet, daß man

Pyrrole der allgemeinen Formel (XI)

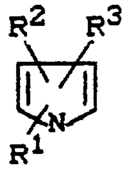

(XI)

in welcher
$R^1$, $R^2$ und $R^3$ die oben angegebene Bedeutung haben,
in inerten Lösemitteln in Anwesenheit von Hilfsstoffen mit N,N-Dialkylaminoacrolein der Formel (XII)

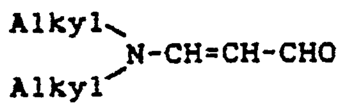

(XII)

wobei
Alkyl für einen geradkettigen oder verzweigten Kohlenwasserstoffrest mit 1 bis 6 Kohlenstoffatom steht, umsetzt.

11. Arneimittel enthaltend disubstituierte Pyrrole nach Anspruch 1.

12. Arzneimittel nach Anspruch 11, dadurch gekennzeichnet, daß es 0,5 - 98 Gew.-% an disubstituierten Pyrrolen bezogen auf die Gesamtmischung enthält.

13. Verwendung von disubstituierten Pyrrolen zur Behandlung von Krankenheiten.

14. Verwendung von disubstituierten Pyrrolen nach Anspruch 1 zur Herstellung von Arzneimitteln zur Behandlung von Krankheiten.

15. Verwendung nach Anspruch 14, zur Behandlung von Hyperlipoproteinämie, Lipoproteinämie oder Arteriosklerose.

Europäisches Patentamt

# EUROPÄISCHER RECHERCHENBERICHT

Nummer der Anmeldung

EP 89 10 4381

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 221 525 (SANDOZ)<br>* Ansprüche 1,9 *<br>----- | 1,15 | C 07 D 207/337<br>C 07 D 207/333<br>A 61 K 31/40 |
| | | | **RECHERCHIERTE SACHGEBIETE (Int. Cl.4)** |
| | | | C 07 D 207/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 16-06-1989 | CASADO Y MARTIN DE MERCA |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)